# EUROPEAN PATENT SPECIFICATION

(11) **EP 3 845 222 B1**
(45) Date of publication and mention of the grant of the patent: **15.03.2023**
(21) Application number: 20216167.5
(22) Date of filing: 21.12.2020
(51) Int. Cl.: A61K 9/20

(54) **MICROENCAPSULATED FORMULATION COMPRISING DIOSMIN, TROXERUTIN AND COUMARINS, COMPOSITIONS THEREOF AND USE THEREOF FOR THE TREATMENT OF CIRCULATORY DISORDERS**
MIKROVERKAPSELTE FORMULIERUNG MIT DIOSMIN, TROXERUTIN UND CUMARINEN, ZUSAMMENSETZUNGEN DAVON UND VERWENDUNG DAVON ZUR BEHANDLUNG VON KREISLAUFERKRANKUNGEN
FORMULATION MICROENCAPSULÉE COMPRENANT DE LA DIOSMINE, DE LA TROXÉRUTINE ET DES COUMARINES, LEURS COMPOSITIONS ET LEUR UTILISATION POUR LE TRAITEMENT DES TROUBLES CIRCULATOIRES

(30) Priority: 19.12.2019 IT 201900024904
(43) Date of publication of application: 07.07.2021
(73) Proprietor: Pharma Line S.r.l., 20154 Milano (IT)
(72) Inventor: Pezzini, Pietro, 46100 Mantova (MN) (IT)
(74) Representative: Hoffmann Eitle

(56) References cited:
- Pharma Line S.R.L.: "Venolen Plus", , 18 October 2019 (2019-10-18), XP055729733, Retrieved from the Internet: URL:https://www.pharma-line.it/sites/defau lt/files/2020-06/Venolen%20plus_IT_FI01%20 18-10-19.pdf [retrieved on 2020-09-10]
- First communication to the Italian Ministry of the notification of releasing the product "Venolen plus compresse rivestite" on the italian market.
- Technical data sheet of the commercial product "Venolen plus"

## Description

The present invention relates to a microencapsulated formulation with a coating matrix comprising at least one lipid, preferably a phospholipid, wherein said formulation comprises or, alternatively, consists of: (a) diosmin, (b) troxerutin and (c) coumarins or an extract of plant species comprising coumarins. Furthermore, the present invention relates to compositions comprising said microencapsulated formulation and, optionally, (d) vitamin C and/or at least one acceptable pharmaceutical or food grade additive. Lastly, the present invention relates to the use of said microencapsulated formulations and compositions thereof for the treatment of disorders of the circulatory system, preferably of a chronic venous insufficiency or of haemorrhoidal disease or edematous fibrosclerotic panniculopathy (PEFS) or lymphedema or trauma or an endothelial dysfunction, and diseases, symptoms or disorders related thereto.

The term "chronic venous insufficiency" (in short, CVI or chronic venous disease, in short CVD) indicates a condition of altered venous return, which sometimes causes discomfort in the lower limbs and skin changes.

The causes of chronic venous insufficiency are the diseases that determine venous hypertension, usually through venous damage or incontinence of venous valves, as it occurs (for example), after deep vein thrombosis. Diagnosis is based on medical history, objective examination and duplex echo-doppler. Treatment comprises compression, wound care and, rarely, surgery. Subjective symptoms are: the sensation of heaviness, tension in the legs, burning sensation, itching. Objective symptoms: presence of varices, capillaries, swelling, oedema, spots, all or variously related.

In the context of the present invention, the terms "chronic venous insufficiency (CVI)" and "chronic venous disease (CVD)" are synonyms and used interchangeably.

The weakness of the venous walls and the incompetence of the venous valves affect each other, resulting in venous hypertension, reflux and stasis. This determines the expression of factors that cause leukocyte adhesion and it represents a significant inflammatory stimulus. Inflammation mediators such as prostaglandins (PGE2, PGF2alpha), thromboxanes (TXBA), histamine, and free radicals are produced as a result of activation of the inflammatory process. Prostaglandins cause increased vessel permeability. Histamine, contained in mast cells, is released following trauma or inflammation, causing arteriolar vasodilation, vasoconstriction of the local venous region and further increasing capillary permeability. Free radicals also cause increased vessel permeability and act as a chemotactic recall for specialised cells such as granulocytes, macrophages, lymphocytes. Furthermore, weakness of venous walls generates oedema, which in turn enhances inflammatory processes.

Furthermore, in oxidative stress conditions, endothelial production of prostacyclin is inhibited and the metabolism of arachidonic acid is diverted toward the synthesis of thromboxane (TX) and of other vasoconstrictor/pro-aggregants eicosanoids. In these pathological situations, the excess of superoxide ions allows the metabolic pathway of arachidonic acid to be directed toward the production of thromboxanes with other substances that activate the TP receptor and carry out platelet pro-aggregant, pro-inflammatory and vasoconstrictive action. These effects are enhanced by the fact that free radicals increase the stability and density of TP receptors. As a result, a vicious circle is generated which determines a pathological remodelling of the wall of the vessels, valves and surrounding tissues. Therefore, chronic venous insufficiency (CVI) progressively worsens and affects the skin too.

Chronic venous insufficiency affects up to 5% of the population in the United States.

The term "lymphedema" is used to indicate the oedema of a limb due to lymphatic hypoplasia (primitive) or obstruction or destruction (secondary) of lymphatic vessels. The symptoms are represented by hard oedema that cannot be compressed, of fibrous texture, in one or more limbs.

Haemorrhoids are vascular structures of the anal canal that play a significant role in maintaining faecal continence. When swollen or inflamed, they cause a syndrome known as haemorrhoidal disease. In particular, the increase in pressure in the veins of the anus-rectal area leads to haemorrhoids. This pressure can result from pregnancy, frequent lifting of heavy loads, or repeated stress during defecation (for example, due to constipation).

Edematous fibrosclerotic panniculopathy (in short, PEFS), commonly referred to as cellulitis, is a degenerative disease of dermal and hypodermic tissue that evolves in stages initially characterised by oedema and subsequently by fibrosis and sclerosis.

The expression endothelial dysfunction is used to indicate an alteration of the normal endothelial function, which entails the loss of some structural and/or functional characteristics. The expression "endothelial activation" is instead used to indicate the acquisition - by the endothelium - of new properties, such as the expression of adhesive molecules during inflammation. There are numerous factors which can cause functional damage to the endothelium and most of them are represented by cardiovascular risk factors.

The drugs or methodologies used to date for the treatment of the aforementioned diseases and disorders or symptoms related thereto are not always effective (for example due to poor gastrointestinal absorption if administered through the oral route), or, they lead to only partial or only temporary resolution of the disorder.

Each tablet of the product "Venolen^{®} plus" comprises micronized diosmin (300 mg), troxerutin (300 mg) and a dry extract of horse chestnut standardized in total coumarins (100 mg). The product "Venolen^{®} plus" is a product which is not microencapsulated with a lipid and which is available on the market since 2011.

Thus, there is a high need to provide compositions or formulations which are highly effective as vasoactive agents and, therefore, that can be used in the preventive and/or curative treatment of disorders of the circulatory system, in particular chronic venous insufficiency (CVI), haemorrhoidal disease, edematous fibrosclerotic panniculopathy (PEFS), lymphedema, trauma and/or endothelial dysfunction, and diseases, disorders or symptoms related thereto.

Furthermore, the need is felt to provide innovative compositions or formulations for an effective preventive and/or curative treatment of the aforementioned diseases capable of allowing a rapid and almost total restoration of the functionality of the venous circulation, in particular of the lower limbs, and of the lymphatic system, that can be used by all the classes of subjects (such as, sportsmen/sportswomen, pregnant or breastfeeding women, the elderly and paediatric subjects, as well as adults in a normal health condition) and devoid of significant side effects.

Lastly, the need is felt to provide compositions or formulations per an effective preventive and/or curative treatment of the aforementioned diseases that are easy to prepare and cost-effective.

Following extensive research and development activity, the Applicant addresses and solves the aforementioned needs by providing a formulation microencapsulated or coated with at least one lipid, preferably a phospholipid, wherein said formulation comprises or, alternatively, consists of: (a) diosmin; (b) troxerutin and (c) coumarins or an extract of a plant species titrated in coumarins (in short, microencapsulated formulation of the invention or formulation of the invention), and compositions comprising said microencapsulated formulation and, optionally, (d) vitamin C and/or at least one additive/excipient of an acceptable pharmaceutical or food grade (in short, compositions of the invention).

The microencapsulated formulations and compositions thereof subject of the present invention are highly effective in the curative and/or preventive treatment of disorders of the circulatory system, in particular chronic venous insufficiency (CVI) (or chronic venous disease, CVD) or haemorrhoidal disease or edematous fibrosclerotic panniculopathy (PEFS) or lymphedema or trauma or endothelial dysfunction and of the diseases, disorders or symptoms related thereto (e.g., lower limb problems such as oedema, walking, burning sensation, itching, paraesthesia, sensation of heaviness, postphlebitic syndrome) and improvement treatments with respect to treatments with products currently available in the prior art.

The scope of the invention is defined by the claims.

An object of the present invention is to provide innovative formulations of the active components, such as (a) diosmin, (b) troxerutin and (c) coumarins, and compositions thereof which are capable of making the administration of said active components highly effective, in particular administration through the oral route following an increase in the gastrointestinal absorption of said active components (or at least some of them) and, therefore, blood bioavailability thereof, with ensuing decrease in the subjective variability to the effectiveness of the composition.

In the context of the present invention, the terms gastrointestinal absorption and intestinal absorption are used interchangeably.

Said increase in the gastrointestinal absorption of the active components (a), (b) and (c) and/or the increase in the blood bioavailability thereof is assumed to be due to the insertion thereof into a coating matrix based on at least one lipid, preferably a phospholipid, which provides gastroprotection to the acidic environment and/or increase in the ability of all or part of the microencapsulated active components, such as (a), (b) and (c), to pass through the intestinal barrier (increased intestinal permeability).

Said high efficacy of the microencapsulated formulations and compositions thereof subject of the present invention and/or said increased blood bioavailability of the active components of said microencapsulated formulations results in a decrease in the effective dose to be administered to a subject in need, with respect to the products of the prior art. As a result, the microencapsulated formulations or compositions thereof of the present invention can therefore have an economic advantage and/or potentially fewer undesired effects, with respect to the products of the prior art.

Furthermore, said high efficacy of the microencapsulated formulations and compositions thereof subject of the present invention and/or said increased blood bioavailability of the active components of said microencapsulated formulations results in achieving a greater effect considering the same daily intake of said microencapsulated formulations or compositions thereof subject of the present invention administered to a subject in a need, with respect to the products of the prior art.

Said high efficacy of the microencapsulated compositions and formulations subject of the present invention and/or said increased blood bioavailability of the active components thereof also results in a decrease in the onset of said compositions and formulations after administration to a subject in need, with respect to the products of the prior art.

In the context of the present invention, the term "*onset*" is used to indicate the latency time between the administration of a composition/product and the pharmacological effect thereof. In other words, onset is the time that elapses from when the composition/product is administered up to the partial or total pharmacological effect.

In addition, the high efficacy of the microencapsulated compositions and formulations subject of the present invention are due to the selected and specific combination of the active components present in the microencapsulated compositions or formulations and/or to the synergy of said active components, with particular reference to (a) diosmin, (b) troxerutin, (c) coumarins and, optionally, (d) vitamin C.

The efficacy of the medical treatment resulting from said specific combination/synergy of the active components (a), (b) and (c) and, optionally, (d) present in the microencapsulated compositions and formulations of the present invention is enhanced by the ability of lipid microencapsulation to increase the gastrointestinal absorption thereof after oral ingestion and, therefore, the blood bioavailability thereof (of all or part of the active components).

In particular, the combination and/or synergy of the active components present in the microencapsulated compositions and formulations subject of the present invention and/or the high blood bioavailability thereof after oral administration, make said compositions and formulations particularly effective in preventing the diseases/disorders/symptoms subject of the present invention (i.e. CVI, PEFS, haemorrhoidal disease, lymphedema, trauma and/or endothelial dysfunctions), in treating the acute stages thereof, and in preventing their recurrence thereof (or relapse) and, therefore, the chronicization thereof.

As a matter of fact, both diosmin and troxerutin are capable of performing actions of increasing venous tone, decreasing capillary permeability, improving hemorheology and tissue perfusion and anti-edematous action, that is to say they are capable of performing a phlebotropic, capillarotropic and vasoprotective action. Coumarins or extracts of a plant species comprising coumarins enhance the microcirculation function. Vitamin C performs antioxidant action and supports the physiological formation of collagen for the normal functioning of blood vessels.

The term "phlebotropic" is used to indicate a compound or drug which, in the context of the treatment of CVI, combines the phlebotonic action (on venous tone) with an enhancement in lymphatic drainage and microcirculation.

Furthermore, the microencapsulated compositions or formulations subject of the present invention are stable over time from the chemical-physical, shelf-life and organoleptic point of view; advantageously, they are more stable than the products of the prior art.

Furthermore, the microencapsulated compositions and formulations of the present invention do not have significant side effects, they are well tolerated and, therefore, can be administered to all subjects, in particular also to pregnant or breastfeeding female subjects, subjects in paediatric age, sportsmen/sportswomen and the elderly.

Lastly, the microencapsulated compositions and formulations of the present invention are easy to prepare and cost-effective.

These and other objects which will be clear from the detailed description that follows, are attained by the microencapsulated formulations and by the compositions of the present invention due to the technical characteristics claimed in the attached claims.

### FIGURES

Figure 1 is a graphical representation of microcapsule structures of the microencapsulated formulation comprising (a), (b) and (c) subject of the present invention.
Figure 2 represents the diffusion of the compounds under analysis (microencapsulated formulation (line with circles) and non-microencapsulated formulation (line with squares) through the Franz diffusion cell apparatus over 24 hours.
Figures 3A and 3B represent the role of the compound under analysis (microencapsulated formulation) on the ATP content and on the PLA2 activity in HUVECs (***p <0.001 with respect to the control under normoxia; ^{##}p <0.01 with respect the control under hypoxia).
Figure 4 represents the result of the ex vivo evaluation test of the contraction of mesenteric vessels (*** p <0.001 with respect to the control).
Figure 5 represents a histopathological examination, through haematoxylin and eosin staining, of the ligated saphenous vein (LSV) in mice treated with the composition according to the invention or Simvastatin (ND, not detectable; ***p <0.001 with respect to carrier and ^{###}p <0.001 with respect to LSV).
Figure 6 represents the immunohistochemical analysis for the VEGF of the ligated saphenous vein (LSV) in mice treated with the composition according to the invention or Simvastatin (ND, not detectable; ***p <0.001 with respect to carrier, ^{###}p <0.001 with respect to LSV).
Figures 7A and 7B respectively represent the quantification of IL-1β and TNF-α cytokines using the ELISA kits to test the ligated saphenous vein (LSV) in mice treated with the composition according to the invention or Simvastatin (ND, not detectable; **p <0.01 with respect to carrier, ^{#}p <0.05 with respect to LSV).

### SUMMARY OF THE INVENTION

In a first aspect, the present invention relates to a formulation comprising (a) diosmin, (b) troxerutin and (c) coumarins microencapsulated with a coating matrix comprising a lipid.

In a second aspect, the present invention relates to a composition comprising said microencapsulated formulation.

In a third aspect, the present invention relates to said microencapsulated formulations and compositions thereof for use as medicament, such as for use in a method for the preventive or curative treatment of disorders of the circulatory system.

In a fourth aspect, the present invention relates to a method for the preventive or curative treatment of disorders of the circulatory system, wherein said method provides for the administration of said microencapsulated formulation or compositions thereof to subjects in need in a therapeutically effective amount.

In a fifth aspect, the present invention relates to the cosmetic (or non-therapeutic) use of said microencapsulated formulations and compositions thereof.

In a sixth aspect, the present invention relates to a process for preparing said microencapsulated formulation (microencapsulation method).

In a seventh aspect, the present invention relates to a microencapsulated formulation obtained using the microencapsulation method of the invention.

### DETAILED DESCRIPTION OF THE INVENTION

Forming an object of the present invention is a microencapsulated formulation (in short, formulation of the invention or microencapsulated formulation of the invention) with a coating matrix, wherein said formulation comprises or, alternatively, consists of:
(a) diosmin;
(b) troxerutin; and
(c) coumarins or an extract of a plant species comprising coumarins, preferably extract of European horse chestnut *(Aesculus hippocastanum* L.) comprising coumarins;
wherein said coating matrix comprises or, alternatively, consists of at least one lipid, preferably at least one phospholipid and, optionally, at least one acceptable pharmaceutical or food grade additive and/or excipient.

In the context of the present invention, with reference to the formulation subject of the invention, the terms "microencapsulated" or "coated" or "covered" or "embedded" are synonyms that can be used interchangeably. For the sake of brevity, in the context of the present invention we will mention only the term "microencapsulated formulation".

Preferably, the formulation of the invention is in solid state.

Diosmin (IUPAC Name 5-hydroxy-2-(3-hydroxy-4-methoxyphenyl)-7-[(2S,3R,4S,5S,6R)-3,4,5-trihydroxy-6-[[(2R,3R,4R,5R,6S)-3,4,5-trihydroxy-6-methyloxan-2-yl]oxymethyl]oxan-2-yl]oxychromen-4-one, common name 5,7,3'-trihydroxy-4'-methoxyflavone, example of CAS No. 520-27-4) is a flavonoid of plant origin contained in citrus fruits and fruits of plant species belonging to the family of *Rutaceae,* which can be obtained by extraction from said plant species, or, by synthetic route starting from hesperidin. Hesperidin is a glycosylated flavanone (example of CAS No. 520-26-3), a type of flavonoid found especially in citrus fruits, predominantly lemons and oranges.

Advantageously, said (a) diosmina comprised in the microencapsulated formulation of the invention, together with (b) and (c), is micronized diosmin, preferably micronized diosmin obtained from hesperidin, more preferably micronized diosmin obtained from hesperidin and comprising a buffer system. An example of (a) diosmin that can be used in the present invention is micronized diosmin having the technical characteristics of the commercial product µSMIN^{®} Plus, as reported in the Examples section. In µSMIN^{®} Plus, the presence of a buffer system (for example, a bicarbonate) prevents the excessive increase in pH, which would determine the ionisation of the molecule and therefore the decrease in the intestinal absorption thereof.

Advantageously, said (a) micronized diosmina comprised in the microencapsulated formulation of the invention, together with (b) such as (b.i) or (b.ii) and (c) such as (c.i), can be obtained by means of methods and equipment known to the man skilled in the art. The powders or granules are defined as "micronized" when they have an average size (or average particle size) comprised in the range from about 10 µm to about 0.5 µm (for example 1 µm, 2 µm, 3 µm, 4 µm, 5 µm, 6 µm, 7 µm, 8 µm, or 9 µm). Said micronized diosmin (a) can be obtained, for example, by mixing diosmin and excipients and subsequent granulation by means of micronization. "Micronization" is defined as a grinding (dry or wet) of coarse solid material, obtaining particles with dimensions smaller than 10 µm. Micronization is carried out, for example, in mills called micronizers (e.g. with vertical elliptical chamber, with opposite or spiral jets) which operate with air flow; they are also called fluid energy mills.

Troxerutin is a flavonol, in particular it is a tri(hydroxyethyl)rutoside (C₃₃H₄₂O₁₉, chemical name 3',4',7'-triO-(β-hydroxyethyl), example of CAS No. 56764-99-9) belonging to the family of bioflavonoids (gamma-benzopyrones). Troxerutin can be obtained by extraction from a plant source or by synthesis.

Troxerutin can be extracted from portions of *Styphnolobium japonicum* (L.) (or Japanese pagoda), such as roots, bark and/or aerial parts, by means of a standard method known to the man skilled in the art, for example, by means of hydroalcoholic extraction (for example, ethanol/water) and subsequent spectroscopic analysis (for example, HPLC).

Advantageously, said (b) troxerutin comprised in the microencapsulated formulation of the invention, together with (a) and (c) such as (c.i), is (b.i) an extract of *Styphnolobium japonicum* titrated in troxerutin, preferably titrated in troxerutin in a percentage by weight with respect to the total weight of the extract comprised in the range from 60% to 99.5% with respect to the total weight of the extract (afor example 65%, 70%, 75%, 80%, 85%, 90%, or 95%), preferably from 70% to 98%, more preferably from 75% to 95% (for example about 80%); more preferably an extract or dry extract of flowers and/or leaves of *Styphnolobium japonicum.*

An example of said (b.i) extract of *Styphnolobium japonicum* titrated in troxerutin is the commercial product "Troxeruside DAB 199" (Nutraceutica s.r.l., Italy) having the technical characteristics as reported in the Examples.

Alternatively, said (b) troxerutin comprised in the microencapsulated formulation of the invention, together with (a) and (c) such as (c.i), is (b.ii) troxerutin obtained by synthetic route (in short, synthetic troxerutin); preferably it is troxerutin obtained by synthetic route using rutin as starting material (for example rutin trihydrate, alternatively referred to as rutoside trihydrate).

For example, said synthetic troxerutin (b.ii) can be obtained by means of the following process: loading purified water, caustic soda and rutoside trihydrate into a reactor. Heating the reaction mass, purging ethylene oxide, cooling and adjusting the pH with dilute sulfuric acid to the predefined limit. Mixing the reaction mass, adjusting the pH with dilute caustic solution to the predefined limit, filtering the reaction mass and transferring the transparent filtrate to another reactor. The reaction mass was heated and distilled off from the water. Adding methanol and filtering the reaction mass and transferring the transparent filtrate to another reactor. Adding isopropyl alcohol, cooling, centrifuging and washing with cold methanol: isopropyl alcohol (IPA) to obtain a wet cake. Loading methanol into a reactor: IPA and the wet cake and heating to reflux. Cooling, centrifuging and washing with methanol: IPA to obtain a wet cake. Loading the wet cake into an IPA reactor and heating to reflux for 30 minutes. Cooling, centrifuging and washing with IPA. Drying and grinding to obtain troxerutin.

The European horse chestnut or horse chestnut *(Aesculus hippocastanum* L., 1753) is a tree belonging to the family *Sapindaceae.*

Advantageously, said (c) extract of a plant species comprising coumarins (c.i) extract of European horse chestnut *(Aesculus hippocastanum)* comprising coumarins, wherein said (c.i), comprised in the microencapsulated formulation of the invention, together with (a) and (b) such as (b.i) or (b.ii), is an extract or dry extract of European horse chestnut bark titrated in total coumarins in a percentage by weight comprised in the range from 1% to 40% with respect to the total weight of the extract (for example 5%, 10%, 15%, 20%, 25%, 30%, or 35%), preferably from 10% to 30%, more preferably from 15% to 25% (for example about 20%).

An example of said (c.i) extract of European horse chestnut *(Aesculus hippocastanum)* titrated in coumarins is the commercial product "Ippocastano Estratto 20%" (NVH Italia srl, Italy) having the technical characteristics as reported in the Examples.

Said (c.i) extract of European horse chestnut *(Aesculus hippocastanum)* comprising coumarins, comprised in the microencapsulated formulation of the invention, can be obtained by extraction of portions of *Aesculus hippocastanum* L., such as roots, bark and/or aerial parts, by means of a standard method known to the man skilled in the art, for example by means of hydroalcoholic extraction (for example, water/ethanol) and subsequent spectroscopic analysis (for example HPLC).

Alternatively, said component (c) extract of a plant species titrated in coumarins comprised in the microencapsulated formulation of the invention, together with (a) and (b) such as (b.i) or (b.ii), can be an extract of a species of plant origin other than European horse chestnut, such as for example, an extract of at least one plant belonging to the botanical family of *Rutaceae, Apiaceae* or Fabaceae, preferably an extract of yellow sweet clover (*Melilotus officinalis* (L.) Pall.) comprising coumarins.

Coumarins are a class of compounds derived from coumarin (IUPAC name: 1-benzopyran-2-one).

In a preferred embodiment of the microencapsulated formulation of the present invention, said formulation comprises or, alternatively, consists of:
(a) micronized diosmin; preferably micronized diosmin obtained from hesperidin by the synthetic route; more preferably micronized diosmin obtained from hesperidin by the synthetic route comprising a buffer system (for example, the commercial product µSMIN^{®} Plus);
(b) troxerutin, preferably (b.i) extract of *Styphnolobium japonicum* comprising troxerutin (preferably titrated from 60% a 99.5%, for example about 85%) or (b.ii) synthetic troxerutin;
(c.i) an extract of European horse chestnut, *Aesculus hippocastanum* L., comprising total coumarins in a percentage by weight comprised in the range from 1% to 40% with respect to the total weight of the extract, preferably from 10% to 30%, more preferably from 15% to 25%, preferably dry extract from bark of European horse chestnut titrated in coumarins as reported above;
and wherein said coating matrix which microencapsulates the formulation of (a), (b) and (c.i) comprises or, alternatively, consists of at least one lipid or phospholipid, preferably a phosphatidylcholine or lecithin (E322), and, optionally, at least one acceptable pharmaceutical or food grade additive and/or excipient, for example silicone dioxide and/or hydroxypropyl cellulose.

The microencapsulated formulation of the present invention may comprise or, alternatively, consist of:
(a) diosmin, preferably micronized diosmin (for example, µSMIN^{®} Plus);
(b) troxerutin, preferably (b.i) extract of *Styphnolobium japonicum* titrated in troxerutin (preferably from 60% to 99.5%, for example about 85%) or (b.ii) synthetic troxerutin;
(c) coumarin, preferably (c.i) an extract of *Aesculus hippocastanum* titrated in total coumarins from 1% to 40% (for example about 20%);
and wherein said coating matrix which microencapsulates the formulation of (a), (b) and (c.i) comprises or, alternatively, consists of a lecithin (E322), such as a sunflower, corn or soy lecithin (preferably sunflower), and, optionally, at least one acceptable pharmaceutical or food grade additive and/or excipient.

The microencapsulated formulation of the present invention may comprise or, alternatively, consist of:
(a) diosmin, preferably micronized diosmin;
(b.i) an extract of *Styphnolobium japonicum* titrated in troxerutin (preferably from 60% to 99.5%, for example about 85%);
(c.i) an extract of *Aesculus hippocastanum* titrated in total coumarins from 1% to 40% (for example about 20%);
And wherein said coating matrix which microencapsulates the formulation of (a), (b) and (c.i) comprises or, alternatively, consists of a lecithin (E322), such as a sunflower, corn or soy lecithin (preferably sunflower), and, optionally, at least one acceptable pharmaceutical or food grade additive and/or excipient.

The microencapsulated formulation of the present invention may comprise or, alternatively, consist of:
(a) diosmin, preferably micronized diosmin (for example, µSMIN^{®} Plus);
(b.ii) synthetic troxerutin;
(c.i) an extract of *Aesculus hippocastanum* titrated in total coumarins from 1% to 40% (for example about 20%);
and wherein said coating matrix which microencapsulates the formulation of (a), (b) and (c.i) comprises or, alternatively, consists of a lecithin (E322), such as a sunflower, corn or soy lecithin (preferably sunflower), and, optionally, at least one acceptable pharmaceutical or food grade additive and/or excipient.

In an embodiment of the present invention, said at least one lipid, comprised in the coating matrix which microencapsulates the formulation comprising (a), (b) and (c) according to any one of the embodiments described in the present invention, is a lipid of plant origin, preferably at least one phospholipid, more preferably at least one phosphoglyceride, even more preferably at least one phosphoglyceride selected from 1,2-diacyl-phospholipids, 1-alkyl-2-acyl-phospholipids, 1-alkenyl-2-acyl-phospholipids; even more preferably at least one diacyl-phospholipid selected from the group comprising or, alternatively, consisting of: phosphatidylcholine or lecithin (E322), phosphatidylserine, phosphatidylethanolamine, phosphatidylinositol, phosphatidic acid and mixtures thereof; of said group preferably phosphatidylcholine or lecithin (E322), advantageously allergen free and/or powdered or granular; more preferably sunflower, corn or soy lecithin (E322); more preferably allergen free powdered or granular lecithin (E322).

Phospholipids are phosphate-containing lipids. The molecules of this class of organic compounds have a water-soluble polar head (i.e., water-soluble and insoluble in apolar solvents) based on phosphate and a hydrophobic non-water-soluble apolar tail (i.e., water-insoluble and soluble in apolar solvents), for this reason they are referred to as amphipathic molecules.

Phosphoglycerides (also called glycerophospholipids) represent the most important class of phospholipids.

Glycerophospholipids (or phosphoglycerides) are all derived from sn-glycerol-3-phosphate, in which glycerol (CH₂OH-CHOH-CH₂OH) is esterified in position 3 with orthophosphoric acid (H₃PO₄). In glycerophospholipids glycerol is esterified in position 2 with a fatty acid, whereas different classes of compounds can be bound in position 1; since carbon 2 is asymmetric, there are two possible stereoisomers: L and D. In nature glycerophospholipids all belong to the L series.

Depending on the nature of the molecule that binds to the position 1 of glycerol, three subclasses of phosphoglycerides can be distinguished: 1,2-di-acyl-phospholipids, 1-alkyl-2-acyl-phospholipids, 1-alkenyl-2-acyl-phospholipids.

Diacyl-phospholipids (phosphoglycerides) derive from the structure of triglycerides, where a fatty acid is replaced by a phosphate group which gives the molecule a negative charge and therefore polarity; this molecule has the generic name phosphatide. A more complex organic molecule, generally serine, choline, ethanolamine, inositol or a single hydrogen atom, is bound to the phosphate group through an ester bond, giving rise to a phospholipid called phosphatidylserine, phosphatidylcholine (or lecithin), phosphatidylethanolamine, phosphatidylinositol or phosphatidic acid, respectively.

Diacyl-phospholipids are characterised by a water-soluble polar head, which dissolves well in water, while the two saturated fatty acids represent the two apolar tails, which are not water-soluble but lipophilic.

The term lecithin refers to a class of chemical compounds present in animal and plant tissues (in particular in the egg yolk).

From a chemical point of view, a lecithin is a phosphatidylcholine ((R)-1-oleoyl-2-palmitoyl-phosphatidylcholine) or, alternatively, a lecithin comprising phosphatidylcholine as the primary component. A phosphatidylcholine is a phosphoglyceride in which phosphatidic acid is esterified with choline. Phosphatidic acids represent the simplest phosphoglycerides, formally produced by the esterification of glycerol in position 1 and 2 with fatty acids and in position 3 with orthophosphoric acid.

Lecithin is a natural emulsifier due to its chemical/physical properties, and it has a secondary antioxidant function, given that it is rich in natural antioxidant substances.

A lecithin E322 is a food additive (emulsifier).

The term "E322" defines that lecithin is a food additive permitted by European legislation and regulated by the Italian Ministerial Decree D.M.1996.

Directive 2008/84/EC of 27 August 2008 (published in the Official Journal of the European Union No L253) lays down the purity criteria which a lecithin must meet in order to be considered to meet food quality standards (lecithin E322): insoluble in acetone (basically the active part of lecithin):: 60% min.; moisture: 2% max.; acidity number: 35 max.; peroxides number: 10 max.; insoluble in toluene (the impurities basically): 0.3% max.

The term "allergen free" means that it does not have any allergen residues.

In an alternative embodiment, said at least one lipid comprised in the coating matrix of the microencapsulated formulations of the present invention (comprising (a), (b) such as (b.i) or (b.ii), and (c) such as (c.i)) is a lipid of plant origin selected from the group comprising or, alternatively, consisting of:
- mono-, di- or tri- glycerols esterified with saturated or unsaturated fatty acids (preferably monounsaturated), preferably esterified with saturated fatty acids (i.e. monoglycerides, diglycerides and/or triglycerides), more preferably mono- and di- glycerols esterified with saturated fatty acids with a number of carbon atoms comprised in the range from C16-C18 and/or mono-, di- or tri- glycerols esterified with saturated fatty acids with a number of carbon atoms comprised in the range from C16-C22;
- free saturated fatty acids;
- free unsaturated fatty acids, preferably mono-unsaturated;
- mono-alcohols esterified with saturated or unsaturated fatty acids (preferably monounsaturated), preferably esterified with saturated fatty acids;
- di-alcohols esterified with saturated or unsaturated fatty acids (preferably monounsaturated), preferably esterified with saturated fatty acids;
- sucrose fatty acid esters (alternatively referred to as sucresters), preferably mixtures of sucrose fatty acid mono-, di- or tri-esters, more preferably sucrose esters primarily of stearic acid and/or palmitic acid;
wherein said saturated or unsaturated fatty acids both free and esterified with glycerol or mono-alcohols or di-alcohols or sucrose, have a number of carbon atoms comprised in the range from C12 to C28, more preferably from C14 to C24, for example C16, C18, C20 and/or C22.

For the sake of clarity, in order to attain the object of the present invention, the components (or active components) (a), (b) and (c) of the formulation of the invention, alternatively to the above, can be microencapsulated with a lipid or phospholipid coating matrix in a separate manner (each individual active component or two active components are microencapsulated) and subsequently be comprised in the composition subject of the present invention optionally together with the active component (d) and/or additives/excipients.

A process known to the man skilled in the art can be used as a process for the microencapsulation of the active components (a), (b) and (c) with the lipid coating matrix to prepare the microencapsulated formulation subject the present invention.

Microencapsulation generally refers to technologies that allow to embed one or more compounds into an individualised small particle structure, in a range comprised from 1 µm to 1000 µm (for example 5 µm, 10 µm, 15 µm, 20 µm, 50 µm, 100 µm, 200 µm, 400 µm, 600 µm, or 800 µm). Depending on the process applied, these particles may have different sizes, shapes and specific structures. The advantage of microencapsulation lies in the fact that the core material (in the present case, the formulation comprising (a), (b) and (c)) is completely coated and isolated from the external environment (for example, the acid pH environment of the stomach). Furthermore, microencapsulation potentially does not affect the properties of the materials of the core.

Advantageously, the microencapsulated formulation according to the present invention, comprising the active components (a), (b) and (c) and a coating matrix comprising a lipid (preferably a lecithin), has a particle size (average particle diameter) comprised from 50 µm to 600 µm (for example 100 µm, 200 µm, 300 µm, 400 µm, 500 µm), preferably from 100 µm to 500 µm, more preferably from 150 µm to 400 µm.

In the context of the present invention, microencapsulation is preferably intended to be applied directly to the particles in solid state, as a formulation comprising (a), (b) and (c) or as an individual component (a), (b) and (c).

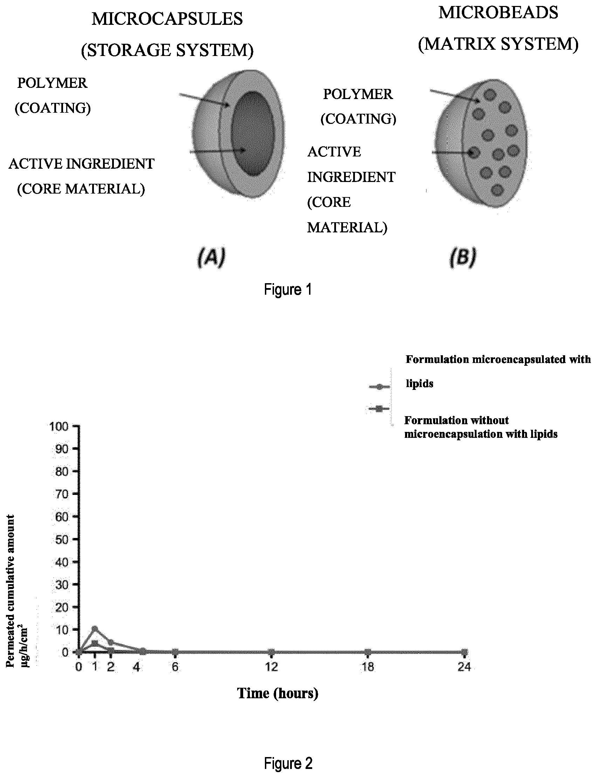

Advantageously, the microencapsulated formulation with lipid matrix comprising (a), (b) and (c) subject of the present invention, according to any one of the embodiments, has one of the following structures (A), (B) or (A+B), as represented in Figure 1 and reported below:
- Core/shell structure (A) (microparticles, microcapsules): the active component or active components (pure or not) is/are confined as a core in one or more shells (single-shell or multi-shell), wherein said shell comprises or, alternatively, consists of the coating matrix.
- Structure of the matrix (B) (microbeads, microparticles): dispersion of the active component or active components (pure or not) in the microencapsulation material (or coating matrix).
- Both structures (A + B) they can be combined to design the core of the matrix surrounded by a shell.

In a preferred embodiment, formulation microencapsulated with lipid matrix comprising (a), (b) and (c) subject of the present invention has said structure (A+B) (or structure of core-shell microparticles dispersed in a matrix), as represented in Figure 1 and reported below: a core/shell structure (A) (microparticles, microcapsules) wherein a mixture of the active components (a), (b) and (c) is confined as a core in one or more shells (single-shell or multi-shell), wherein said shell comprises or, alternatively, consists of the coating matrix according to the invention (i.e. comprising a lipid, preferably a lecithin); furthermore, a plurality of core/shell structures (A) are dispersed in the coating matrix according to the invention (i.e. comprising a lipid, preferably a lecithin), according to said structure (B).

According to a preferred example, in said structure (A+B) (or core-shell microparticles dispersed in a matrix) the microparticles with core/shell structure (A) have a mono-shell.

For example, said microencapsulated formulation of the present invention has a structure comprising or, alternatively, consisting of said coating matrix comprising or, alternatively, consisting of at least one lipid in which core-shell structures are dispersed, wherein said shell comprises or, alternatively, consists of (a) diosmin; (b) troxerutin (for example (b.i) or (b.ii); and (c) coumarins or an extract of a plant species comprising coumarins (for example c.i), and wherein said core comprises or, alternatively, consists of said coating matrix comprising or, alternatively, consisting of at least one lipid.

In an alternative preferred embodiment, the formulation microencapsulated with lipid matrix comprising (a), (b) and (c) subject of the present invention has said structure (A+B) (or structure of core-shell microparticles dispersed in a matrix), as represented in Figure 1 and reported below: three distinct core/shell structures (A) (microparticles, microcapsules) wherein each active component (a), (b) and (c) is independently confined (not mixed) as a core in one or more shells (single-shell or multi-shell), wherein said shell comprises or, alternatively, It consists of the coating matrix according to the invention (i.e. comprising a lipid, preferably a lecithin); furthermore, a plurality of core/shell structures (A) are dispersed in the coating matrix according to the invention (i.e. comprising a lipid, preferably a lecithin), according to said structure (B).

For example, said microencapsulated formulation of the present invention has a structure comprising or, alternatively, consisting of said coating comprising or, alternatively, consisting of at least one lipid in which a plurality of at least three core-shell structures are dispersed, wherein a first shell comprises or, alternatively, consists of (a) diosmin, a second shell comprises or, alternatively, consists of (b) troxerutin; and a third shell comprises or, alternatively, consists of (c) coumarins or an extract of a plant species comprising coumarins, and wherein said core comprises or, alternatively, consists of said coating matrix comprising or, alternatively, consisting of at least one lipid.

In a preferred embodiment, the microencapsulated formulation subject the present invention comprising (a), (b) such as (b.i) or (b.ii) and (c) such as (c.i), and having - as coating matrix - at least one lipid, preferably a phospholipid, for example a phosphatidylcholine or lecithin (E322), is produced by means of a microencapsulation process (in short, microencapsulation process of the present invention) having at least one of the following steps and characteristics:
- Drying, granulation and microencapsulation combined in a single process (3 in 1) with a short drying time (for example from 5 minutes to 180 minutes, for example 10 minutes, 20 minutes, 30 minutes, 60 minutes, 90 minutes or 120 minutes), allowing less physical-chemical stress for the materials to be coated.
- The process can be applied to particles of sizes up to 20 µm (for example from 0.5 µm to 20 µm, such as 1 µm, 5 µm, 10 µm, or 15 µm), as a mixture of (a), (b) and (c) or the individual components (a), (b) and (c).
- Multilayer coating: continuous sequence of individual spraying steps.
- The large surface of the product facilitates the application of the liquid coating or adhesive medium.
- Benefits for the environment, given that spray loss is reduced to the minimum.
- Reliable processing of even highly friable products.
- Efficient coating of complex geometries, as well as products and particles with a hollow structure.
- Even flow conditions in the container allow an even and rapid coating.
- Homogeneous particle size, shape and geometry with almost spherical granules.
- Scalable spray speed allows batches of different sizes.
- Droplet size is stable irrespective of the spray volume.

According to a preferred embodiment, the microencapsulation process of the present invention is performed by means of a fluid bed system, i.e. a microencapsulation system comprising a fluid bed process chamber (for example, a fluid bed chamber according to standard processes and machines). In said fluid-bed chamber the components to be microencapsulated (i.e. a mixture of (a), (b) and (c)) are under flow motion of a gas and, at the same time, the coating matrix of the present invention comprising a lipid is sprayed (emitted by a nozzle by means of spray) into said fluid-bed chamber. The result is the microencapsulation of the active components (a), (b), (c) in the coating matrix to obtain the microencapsulated formulation comprising a lipid according to the invention. Said microencapsulation in a fluid bed is preferably performed at a process temperature comprised from 20°C to 80°C (for example 25°C, 35°C, 40°C, 50°C, 60°C, or 70°C), preferably from 30°C to 60°C.

Advantageously, said fluid-bed microencapsulation process is applied so as to provide the formulation of (a), (b) and (c) microencapsulated in a coating matrix comprising a lipid (for example a lecithin) said microencapsulated formulation having the structure of core-shell microparticles dispersed in a coating matrix (i.e. structure (A+B) according to figure 1).

Forming an object of the present invention is the microencapsulated formulation of the present invention comprising (a), (b) such as (b.i) or (b.ii) and (c) such as (c.i), and having - as coating matrix - at least one lipid, preferably a phospholipid, for example a phosphatidylcholine or lecithin (E322), that can be obtained by means of the microencapsulation process described in the present invention.

The homogeneous lipid coating of the microencapsulation, obtained by means of the processes of the present invention, is suitable to confer gastroprotection and increase of the gastrointestinal absorption of the active components (a), (b) and (c) of the microencapsulated formulation of the present invention, and therefore increase and stabilisation of the blood bioavailability thereof.

The homogeneous lipid coating of the microencapsulation, obtained by means of the processes of the present invention, is suitable for the modified release of the active components (a), (b) and (c) of the microencapsulated formulation of the present invention: delayed release, prolonged release etc. It is also possible to confer modified release characteristics to only one or more selected active components of the formulation.

The homogeneous lipid coating of the microencapsulation, obtained by means of the processes of the present invention, is an effective protection against oxygen and/or moisture, which allows greater durability and also the use of sensitive active components. Furthermore, said microencapsulation isolates the particles allowing to use physically and/or chemically incompatible active components in the same product.

Lastly, the homogeneous lipid coating of the microencapsulation, obtained by means of the processes of the present invention, is suitable for masking the taste of the active components (a), (b) and (c) of the microencapsulated formulation of the present invention in a highly efficient manner which allows administration thereof even in case they have unpleasant taste and/or odour. Furthermore, possible irritant effects of said active components can be reduced.

In an embodiment of the microencapsulated formulation of the present invention, the (i):(ii) weight ratio wherein (i) is the mixture comprising (a), (b) and (c) and (ii) is the coating matrix comprising at least one lipid, wherein both (i) and (ii) are according to any one of the embodiments described in the present invention, is comprised in a range from 24:1 to 3:1, preferably from 18:1 to 6:1, more preferably from 15:1 to 10:1, for example 12 + 1:1.

In an embodiment of the microencapsulated formulation of the present invention, the components are comprised according to the following percentages by weight with respect to the total weight of the formulation (i.e. microencapsulated formulation comprising (a), (b) and (c) and the coating matrix comprising at least one lipid):
- said (a) diosmin, preferably micronized diosmin, more preferably micronized diosmin obtained from hesperidin, more preferably micronized diosmin comprising a buffer system, is comprised in a percentage comprised in the range from 20% to 70%, preferably from 30% to 60%, more preferably from 40% to 50% (for example about 44%-45%);
- said (b.i) extract of *Styphnolobium japonicum* titrated in troxerutin in a range from 60% to 99.5%, preferably from 70% to 98%, more preferably from 75 to 95% (for example ≥ 80%), or, alternatively, (b.ii) synthetic troxerutin, is comprised in a percentage comprised in the range from 10% to 60%, preferably from 20% to 50%, more preferably from 30% to 40% (for example about 35%-36%);
- said (c.i) extract of European horse chestnut, *Aesculus hippocastanum* L., comprising total coumarins in a % by weight comprised in the range from 1% to 40% with respect to the total weight of the extract, preferably from 10% to 30%, more preferably from 15% to 25% (for example about 20%), is comprised in a percentage comprised in the range from 0.5% to 35%, preferably from 1% to 25%, more preferably from 5% to 15% (for example about 11%-12%); equivalent to coumarins as such in a % by weight comprised in the range from 0.05% to 14%, preferably from 0.1% to 10%, more preferably from 0.5% to 6%; and,
- said coating matrix, comprising or, alternatively, consisting of at least one lipid, preferably a phospholipid, more preferably a phosphatidylcholine or lecithin (E322), is comprised in a percentage comprised in the range from 0.1% to 35%, preferably from 0.5% to 25%, more preferably from 1% to 15% (for example about 7% to 8%).

In the microencapsulated formulation of the present invention, the active components (a) diosmin, (b) troxerutin (preferably (b.i) extract of *Styphnolobium japonicum* titrated in troxerutin from 60% to 99.5%, for example about 80%) and (c.i) extract of *Aesculus hippocastanum* titrated in coumarins from 1% to 40% (for example about 20%) are at the following weight ratio with respect to each other [(a) from 30 to 50] : [(b) from 20 to 40] : [(c.i) from 5 to 15], preferably [(a) from 35 to 40] : [(b) from 28 to 32] : [(c.i) from 9 to 11].

Forming an object of the present invention is a composition comprising:
- the formulation, comprising (a), (b) such as (b.i) or (b.ii) and (c) such as (c.i), microencapsulated with a coating matrix comprising a lipid or a phospholipid, according to any one of the embodiments described in the present invention,
- and, optionally, at least one acceptable pharmaceutical or food grade additive and/or excipient (in short, composition of the invention).

In a preferred embodiment, besides said formulation comprising (a), (b) such as (b.i) or (b.ii) and (c) such as (c.i) microencapsulated with a coating matrix comprising a lipid or a phospholipid, said composition subject of the invention further comprises (d) L-ascorbic acid (or ascorbic acid or vitamin C, synonyms of L-ascorbic acid).

In a preferred embodiment, said composition subject of the invention and comprising a microencapsulated formulation comprising or, alternatively, consisting of:
(a) diosmin, preferably micronized diosmine, more preferably micronized diosmin obtained from hesperidin by the synthetic route, still more preferably micronized diosmin obtained from hesperidin by the synthetic route comprising a buffer system (for example, µSMIN^{®} Plus);
(b) troxerutin, preferably (b.i) extract of *Styphnolobium japonicum* comprising troxerutin in a range from 60% to 99.5% with respect to the total weight of the extract, preferably from 70% to 98%, more preferably from 75 to 95%, or, alternatively, (b.ii) synthetic troxerutin;
(c) coumarins or (c.i) an extract of European horse chestnut *(Aesculus hippocastanum* L.) comprising total coumarins in a % by weight comprised in the range from 1% to 40% with respect to the total weight of the extract, preferably from 10% to 30%, more preferably from 15% to 25%, preferably dry extract from bark of European horse chestnut;

wherein said microencapsulated formulation is microencapsulated with a coating matrix comprising or, alternatively, consists of at least one lipid or phospholipid, preferably a phosphatidylcholine or lecithin (E322) and, optionally, additives/excipients such as, for example, silicone dioxide and/or hydroxypropyl cellulose;
and wherein said composition further comprises (d) vitamin C and, optionally, at least one acceptable pharmaceutical or food grade additive and/or excipient (for example, magnesium stearate, silicon dioxide, dicalcium phosphate, microcrystalline cellulose and/or film-forming agents).

The composition subject of the invention may comprise:
the microencapsulated formulation comprising or, alternatively, consisting of:
   (a) diosmin, preferably micronized diosmin (for example, µSMIN^{®} Plus);
   (b) troxerutin, preferably (b.i) extract of *Styphnolobium japonicum* titrated in troxerutin from 60% to 99.5% (for example about 85%), or, alternatively, (b.ii) synthetic troxerutin;
   (c) coumarins, preferably (c.i) an extract of *Aesculus hippocastanum* titrated in coumarins from 1% to 40% (for example about 20%);
wherein said microencapsulated formulation is microencapsulated with a coating matrix comprising or, alternatively, consisting of a lecithin (E322), such as a sunflower, corn or soy lecithin (preferably sunflower), and, optionally, at least one acceptable pharmaceutical or food grade additive and/or excipient; and
wherein said composition further comprises (d) vitamin C and, optionally, at least one acceptable pharmaceutical or food grade additive and/or excipient.

The composition subject of the invention may comprise:
the microencapsulated formulation comprising or, alternatively, consisting of:
   (a) diosmin, preferably micronized diosmin (for example, µSMIN^{®} Plus);
   (b.i) extract of *Styphnolobium japonicum* titrated in troxerutin from 60% to 99.5% (for example about 85%);
   (c.i) an extract of *Aesculus hippocastanum* titrated in coumarins from 1% to 40% (for example about 20%); wherein said microencapsulated formulation is microencapsulated with a coating matrix comprising or, alternatively, consisting of a lecithin (E322), such as a sunflower, corn or soy lecithin (preferably sunflower), and, optionally, at least one acceptable pharmaceutical or food grade additive and/or excipient; and
wherein said composition further comprises (d) vitamin C and, optionally, at least one acceptable pharmaceutical or food grade additive and/or excipient.

The composition subject of the invention may comprise:
the microencapsulated formulation comprising or, alternatively, consisting of:
   (a) diosmin, preferably micronized diosmin (for example, µSMIN^{®} Plus);
   (b.ii) synthetic troxerutin;
   (c.i) an extract of *Aesculus hippocastanum* titrated in coumarins from 1% to 40% (for example about 20%); wherein said microencapsulated formulation is microencapsulated with a coating matrix comprising or, alternatively, consisting of a lecithin (E322), such as a sunflower, corn or soy lecithin (preferably sunflower), and, optionally, at least one acceptable pharmaceutical or food grade additive and/or excipient; and
wherein said composition further comprises (d) vitamin C and, optionally, at least one acceptable pharmaceutical or food grade additive and/or excipient.

In an embodiment of the composition of the invention, the components are comprised according to the following percentages by weight with respect to the total weight of the composition:
- said (a) diosmin, preferably micronized diosmin, more preferably micronized diosmin obtained from hesperidin, more preferably micronized diosmin obtained from hesperidin comprising a buffer system, is comprised in a percentage comprised in the range from 10% to 65%, preferably from 20% to 55%, more preferably from 30% to 45% (for example about 37%-38%);
- said (b.i) extract of *Styphnolobium japonicum* titrated in troxerutin in a range from 60% to 99.5%, preferably from 70% to 98%, more preferably from 75 to 95% (for example ≥ 80%), or, alternatively, (b.ii) synthetic troxerutin, is comprised in a percentage comprised in the range from 5% to 55%, preferably from 20% to 40%, more preferably from 25% to 35% (for example about 29%-31%);
- said (c.i) extract of European horse chestnut, *Aesculus hippocastanum* L., comprising total coumarins in a % by weight comprised in the range from 1% to 40% with respect to the total weight of the extract (preferably from 10% to 30%, more preferably from 15% to 25%), is comprised in a percentage comprised in the range from 0.5% to 35%, preferably from 1% to 25%, more preferably from 5% to 15% (for example about 10%-11%); equivalent to coumarins as such in a percentage by weight comprised in the range from 0.05% to 14%, preferably from 0.1% to 10%, more preferably from 0.5% to 6% (for example about 2%);
- said coating matrix, comprises or, alternatively, consists of at least one lipid, preferably a phospholipid, more preferably a phosphatidylcholine or lecithin (E322), comprised in a percentage comprised in the range from 0.1% to 35%, preferably from 0.5% to 25%, more preferably from 1% to 15% (for example about 6% to 7%);
- said (d) vitamin C (not comprised in the microencapsulated formulation) is comprised in the composition in a % comprised in the range from 0.1% to 25%, preferably from 0.5% to 15%, more preferably from 1% from 10% (for example about 2 or 3%-4%).

The compositions of the present invention, comprising said microencapsulated formulations (i.e. (a)+(b)+(c) + lipid coating matrix) and, optionally, (d), may comprise at least one acceptable pharmaceutical or food grade additive and/or excipient, i.e. a substance devoid of therapeutic activity suitable for pharmaceutical or food use. In the context of the present invention, the acceptable additives and/or excipients for pharmaceutical or food use comprise all the ancillary substances known to the man skilled in the art for the preparation of compositions in solid, semi-solid or liquid form, such as, for example, diluents, solvents (including water), solubilisers, acidifiers, thickeners, sweeteners, flavour enhancers, colorants, sweeteners, lubricants, surfactants, preservatives, stabilisers, pH stabilising buffers and mixtures thereof. Preferably, said at least one acceptable pharmaceutical or food grade additive and/or excipient is/are selected from: magnesium stearate, silicon dioxide, dicalcium phosphate, microcrystalline cellulose and/or film-forming agents.

In an embodiment, besides said microencapsulated formulation (i.e. (a)+(b)+(c) + lipid coating matrix) and, optionally, (d) and/or additives, the composition of the present invention may further comprise at least one further active component selected from the group comprising or, alternatively, consisting of: anti-inflammatory agents, antioxidants, anti-radical agents, nutrients, vitamins of group B, E, D, A , prebiotics, probiotics belonging to the families of yeasts and bacteria, enzymes, immunostimulants, organic and/or inorganic salts, melatonin, plant extracts.

The composition subject of the present invention can be in the form of a liquid, such as solution, biphasic liquid system, dispersions or suspension of a solid in a liquid, semisolid form, such as gel, cream or foam, or in the form of a solid, such as powder, granules, flakes, tablets or capsules.

Preferably, the compositions of the invention are formulated in a form suitable for sublingual, buccal administration through the oral (or gastroenteric) route.

In a preferred embodiment of the invention, the compositions of the invention are administered through the oral route in the form of a solid; advantageously, in the form of a solid or in the form of a mouth-soluble (or mouth-dispersible, that dissolve in the oral cavity) solid, such as powder, granules, flakes, tablets or chewable tablets, capsules or soft-gel, preferably tablets or capsules.

When the compositions of the invention are in the form of solid for oral use, preferably tablets or granules, said solid forms may be coated with gastroprotective films.

In a further embodiment of the invention, the compositions of the invention are administered through the oral route in the form of a liquid, such as solution, biphasic liquid system, dispersions or suspension of a solid in a liquid (for example to be prepared at the time of use).

In an alternative embodiment of the invention, the compositions of the invention are in a form suitable for administration through the topical route, preferably oral topical (oral cavity) or cutaneous topical or rectal topical administration, for example oral sprays, skin sprays, oral gels or creams, skin gels or creams, rectal gels or creams, patches or bandages comprising the composition of the invention; preferably creams or gels.

In a further alternative embodiment of the invention, the compositions of the invention are in a form suitable for administration by injection, preferably through the intravenous or intramuscular or subcutaneous route.

The composition of the invention, comprising said microencapsulated formulation subject the present invention according to any one of the described embodiments, may be a pharmaceutical composition, a medical device composition, a dietary supplement, a food or novel food or nutraceutical composition.

In the context of the present invention, the expression "medical device" is used in the meaning according to the Italian Legislative Decree n° 46 dated 24 February 1997 or according to the new Medical Device Regulation (EU) 2017/745 (MDR).

Forming an object of the present invention are the microencapsulated formulations ((a)+(b)+(c)+lipid matrix) of the present invention and the compositions of the present invention comprising said microencapsulated formulations, according to any one of the described embodiments, for use as medicament.

The microencapsulated formulations and the relative compositions subject of the present invention are vasoactive agents and they act on venous tone, microcirculation, lymphatic system and inflammation of blood and capillary vessels, preferably of the lower limbs or of the anus-rectal area.

Forming an object of the present invention are the microencapsulated formulations comprising (a), (b) and (c) and the compositions of the present invention comprising said microencapsulated formulations and, optionally, (d) and/or food or pharmaceutical grade additives/excipients (according to any one of the embodiments of the present invention) for use in a method for the preventive and/or curative treatment of a disorder of the circulatory system or for use as vasoactive agents, in subjects in need, wherein said treatment method, provides for the administration of an effective dose of a microencapsulated formulation or relative composition subject of the present invention to said subject.

Drugs or vasoactive substances are substances capable of acting on nerve centres for the control of vasal motility (vasoconstriction and vasodilation).

Forming an object of the present invention are the microencapsulated formulations comprising (a), (b) and (c) and the compositions of the present invention comprising said microencapsulated formulations and, optionally, (d) and/or food or pharmaceutical grade additives/excipients according to any one of the embodiments of the present invention, for use in a method for the preventive and/or curative treatment of a chronic venous insufficiency (or chronic venous disease), and diseases, symptoms or disorders related to or deriving from said chronic venous insufficiency, in a subject in need, wherein said treatment method, provides for the administration of an effective dose of a microencapsulated formulation or relative composition subject of the present invention to said subject.

Preferably, said diseases, symptoms or disorders related to or deriving from the chronic venous insufficiency are selected from: oedema (anti-edematous action), difficulty in walking, burning sensation, itchy skin, paraesthesia or formication, pain, muscle cramps, sensation of bloating, sensation of heaviness in the lower limbs, restless legs syndrome, fatigue in the legs, postphlebitic syndrome, stasis dermatitis and couperose skin.

Forming an object of the present invention are the microencapsulated formulations comprising (a), (b) and (c) and the compositions of the present invention comprising said microencapsulated formulations and, optionally, (d) and/or food or pharmaceutical grade additives/excipients, according to any one of the embodiments of the present invention, for use in a method for the preventive and/or curative treatment of the haemorrhoidal disease of edematous fibrosclerotic panniculopathy (PEFS or cellulitis) or of lymphedema (for example, following a conventional treatment of breast cancer, post mastectomy and radiation therapy) or of trauma or of endothelial dysfunction, and of diseases, disorders or symptoms related thereto, in a subject in need, wherein said treatment method, provides for the administration of an effective dose of a microencapsulated formulation or relative composition subject of the present invention to said subject.

Forming an object of the present invention are a method for the preventive and/or curative treatment of a disorder of the circulatory system (as vasoactive agents), chronic venous insufficiency (or chronic venous disease), haemorrhoidal disease, of edematous fibrosclerotic panniculopathy and/or of diseases or symptoms related thereto (for example, as described in the present invention), in a subject in need, wherein said treatment method, provides for the administration of an effective dose of a microencapsulated formulation or relative composition subject of the present invention to said subject.

Forming an object of the present invention are a non-therapeutic use (in a healthy subject) or a cosmetic use of a microencapsulated formulation or relative composition subject of the present invention, in order to maintain or improve the skin tone, to decrease or eliminate telangiectasias or couperose skin or xerosis cutis.

Telangiectasias are unesthetic widening of the blood vessels (particularly on the legs) and not harmful to health.

Couperose is a skin lesion that mainly affects the face (skin blemishes), characterised by red spots due to dilation of the capillaries. Reddening occurs mainly at cheeks, nose wings and chin, and it is more or less evident depending on the extent of the problem.

Xerosis cutis indicates the progressive dryness of the skin: when the amount of water in the stratum corneum drops below 20%, the skin appears arid, dry and dehydrated.

In the context of the present invention, the term "subject/s" is used to indicate human or animal subjects, preferably mammals (e.g. pets such as dogs, cats, horses, sheep or cattle). Preferably, the compositions of the invention are for use in treatment methods for human subjects.

Unless specified otherwise, the expression composition or formulation or other comprising a component at an amount "comprised in a range from x to y" is used to indicate that said component can be present in the composition or formulation or other at all the amounts present in said range, even though not specified, extremes of the range comprised.

Unless specified otherwise, the content of a component in a composition or formulation refers to the percentage by weight of that component with respect to the total weight of the composition or formulation. Unless specified otherwise, the indication that a composition or mixture or other "comprises" one or more components means that other components can be present besides that or those specifically indicated and the indication that a composition or mixture or other "consists" of determined components means that the presence of other components is excluded.

### EXAMPLES

Examples of microencapsulated formulations according to the present invention are reported in Table 1 (for example, product under the trade name alpha-gamma bioactive^{™} manufactured by Pharma Line, Italy).

**Table 1. tab= tablet; d.e.: dry extract. (1) for example: µSMIN^{®} Plus. (2) for example: Troxerutin DAB 199. (3) for example: 20%. European horse chestnut Extract (4) for example: SternPur SP (lecithin). (5) for example: silicon dioxide and/or hydroxypropyl cellulose (HPC).**

| Microencapsulated formulation | mg per tab | supply per tab | % for tab |
|---|---|---|---|
| (a) micronized diosmin⁽¹⁾ | 375 | 300 | 44.65 |
| (b) troxerutin from *Sophora japanica* (I.)⁽²⁾ or synthetic troxerutin | 300 | 300 | 35.70 |
| (c) 20% European horse chestnut d.e. in coumarins⁽³⁾ | 100 | 100 | 11.90 |
| phospholopids⁽⁴⁾ | q.s. | | 7.75 |
| additives and/or excipients⁽⁵⁾ | q.s. | | |
| Total | 839 | | 100 |

- µSMIN^{®} Plus (Giellepi S.p.a. Italy), product having the following technical features: active ingredients: flavonoids NLT 90.0% (UV test), diosmin NLT 80.0%, excipients up to 100%; physical form: hygroscopic granulate; water: NMT 3.0%; bulk density: 330 - 430 g/l; mesh size: up to 600 µm NMT 10%, 100 - 600 µm NLT 75%, through 100 µm NMT 15% (NLT: i.e. "not less than", NMT: i.e. "not more than").
- Troxerutin DAB 199 (Nutraceutica srl, Italia), product having the following technical features: extract of *Styphnolobium japonicum* obtained by hydroalcoholic extraction (ethanol/water); CAS No. 7085-55-4; Molecular weight 742.68; composition: troxerutin (tri(hydroxyethyl)rutoside) >= 80%, others o-(2-hydroxyethyl) <=10%, mono(hydroxyethyl)rutoside <5%, tetra(hydroxyethyl)rutoside <5%; melting point: 168-176°C, residue after calcination (2g / 525°C / 3 hours) >= 0.1%; loss on drying <=5%; sulfuric ash <=0.4%; mesh size 95% 80 mesh; IR identification and chromatographic examination.
- 20% European horse chestnut extract (NVH ITALIA srl, Italy), product having the following technical features: extract of European horse chestnut *(Aesculus hippocastanum)* titrated in coumarins obtained by hydroalcoholic extraction (water/ethanol: 70/30, v/v); composition: Coumarins >= 20% (HPLC), maltodextrins; solvent residue: <= 0.5% (gas chromatography); density (tapped) about 0.5 g/ml; average particle size: >= 90% pass through meshes n° 500 (mesh method Eur. Ph.); loss on drying (105°C/3 hours): <= 5%.
- SternPur SP (Sternchemie, Germany), product having the following technical features: composition: sunflower lecithin (E 322) and silicon dioxide (E 551); fatty acids (%): (C16:0) 14-18, (C18:0) 3-5, (C18:1) 12-14, (C18:2) 62-66, (C18:3) 2-4; insoluble in acetone ≥ 95 %; water ≤ 2 %; peroxide value ≤ 5 meq/kg; acidity value ≤ 35 mg KOH/g.

Examples of compositions according to the present invention are reported in Table 2 and 3.

**Table 2. tab= tablet; d.e.: dry extract. (1), (2), (3), (4) e (5): as defined in Table 1. (6) for example: magnesium stearate, silicon dioxide, dicalcium phosphate, microcrystalline cellulose and/or film-forming agents.**

| Composition | mg per tab | supply per tab | % for tab |
|---|---|---|---|
| Microencapsulated components | | | |
| (a) micronized diosmin⁽¹⁾ | 375 | 300 | 37.5 |
| (b) troxerutin from *Sophora japanica* (I.)⁽²⁾ or synthetic troxerutin | 300 | 300 | 30.0 |
| (c) 20% European horse chestnut d.e. in coumarins⁽³⁾ | 100 | 100 | 10.0 |
| phospholopids(4) + additives and/or excipients⁽⁵⁾ | q.s. | | 6.5 |
| Formulation Total | 839 | | |

| NON-microencapsulated components | | | |
|---|---|---|---|
| (d) vitamin C | 25 or 33.3 | 25 (31%NRV) | 2.5 or 3.3 |
| additives and/or excipients⁽⁶⁾ | 127 | | 12.7 |
| Overall total | 1000 | | 100 |

**Table 3. (1), (2), (3), (4) e (5): as defined in Table 1.**

| Composition | mg per tab |
|---|---|
| Components microencapsulated in lipid matrix (i.e. Microencapsulated formulation) | |
| (a) micronized diosmin⁽¹⁾ | 375 |
| (b) troxerutin from Sophora japanica (I.)⁽²⁾ | 300 |
| (c) 20% European horse chestnut d.e. in coumarins⁽³⁾ | 100 |
| phospholopids⁽⁴⁾ + additives and/or excipients⁽⁵⁾ | q.s. |
| Formulation Total | 839 |

| NON-microencapsulated components in lipid matrix | |
|---|---|
| (d) vitamin C | 25 or 33.3 |
| magnesium stearate | 9.9 |
| silicon dioxide | 9.9 |
| dicalcium phosphate | 48.95 |
| microcrystalline cellulose | 48.95 |
| film-forming agents | 10 |
| Overall total | 1000 |

Possible dosages of the microencapsulated formulation according to Table 1 or of the compositions of the invention according to Table 2 and 3 are as follows:
- CVI: one tablet a day, two tablets a day taken simultaneously (in case of more troublesome symptoms), one tablet twice a day;
- Haemorrhoidal disease: one tablet three times a day as an acute attack dose, then one or two tablets a day as maintenance therapy;
- Lymphedema: one tablet twice a day;
- Trauma: one tablet twice a day as an acute attack dose, then one tablet daily as maintenance therapy;
- PEFS: one tablet twice a day.

Thanks to the endothelium tropism, the venotonic action, the anti-inflammatory and antioxidant capacity, the anti-edematous capacity and the regenerative action of the components (a), (b), (c) and, optionally, (d) with high blood bioavailability due to the microencapsulation thereof in a lipid-based coating matrix, preferably phospholipid, the compositions and formulations subject of the present invention exert the following effects:
- they restore tone to the vessels, thanks to various mechanisms, among which the enhancing of the effect of noradrenaline on the vessels and the protective action on the wall;
- they promote the restoration of the integrity of the endothelium, thanks to various mechanisms;
- they protect vessel endothelium from oxidative insult, both by neutralising free radicals and by reducing the production of free radicals by the activated immune system cells (given that the active components control the activation of immune system cells by removing the causes of such activation);
- they combat the increase in endothelium permeability, which is the basis of the formation of oedema;
- they actively promote the resorption of oedema.

A composition according to the invention (similarly to Table 2 or 3) was marketed under the trade name "Venolen^{®} plus" (tablets) according to the following timetable: first communication to the Italian ministry with notification of the box (not package leaflet) on 13 January 2020; manufacture of the first batch in May 2020; first put on the market on 29 May 2020;

### STUDY DESIGN

Title: Effect of the compositions under analysis in the treatment of chronic venous insufficiency (CVI) in *in vitro* and *in vivo* models.

*In vitro* contraction of mouse veins and mesenteric arteries and *in vivo* venotonic mechanisms provide a convenient medium for simulating all vascular events observed in the CVI and studying the effects of the compositions under analysis on the constriction of varicose veins and arteries and the related pathological effects (pain, oedema, itching and sensation of heaviness in the lower limbs).

### 1. COMPOSITIONS under analysis

- Composition 1: composition according to the present invention comprising:
   - Microencapsulated formulation 1, comprising [(a) micronized diosmin, (b) troxerutin (from extract or synthetic) and (c) dry extract of European horse chestnut *(Aesculus hippocastanum* L.), standardised in coumarins] wherein (a), (b) and (c) are microencapsulated with a coating matrix comprising at least one phospholipid; and
   - (d) Vitamin C, and
   - at least one food or pharmaceutical grade additive and/or excipient.

An example of microencapsulated formulation 1 may be the formulation according to Table 1 and/or the product under the trade name alpha-gamma bioactive^{™} or a product comprising alpha-gamma bioactive^{™}. An example of Composition 1 is the composition according to Table 2 or 3.
- Composition 2: comparison composition, not according to the present invention, comprising (a) micronized diosmin, (b) troxerutin (from extract or synthetic), (c) dry extract of European horse chestnut standardised in coumarins, (d) Vitamin C and at least one food or pharmaceutical grade additive and/or excipient. In Composition 2, components (a), (b) and (c) are not microencapsulated with at least one lipid or phospholipid.

### 2. METHODS

### 2.1 Characterisation

- *In vitro* model of chronic venous insufficiency: effect of the composition under analysis (composition 1 according to the invention) on the hypoxia-induced activation of human endothelial cells.
- *Ex vivo* and *in vivo* models of chronic venous insufficiency: mouse mesenteric vessels and adult NMRI mice will be used for the experiments and composition 1 according to the invention will be analysed.
- Absorption method (permeation study): Franz diffusion cells (comparison between composition 1 according to the invention and comparison composition 2).

### 2.2 In vitro model of chronic venous insufficiency (CVI): effect of the composition under analysis on the hypoxia-induced activation of human endothelial cells.

Activation of the endothelium during blood stasis leads to a cascade of reactions which alter the structure of the venous wall in the long term. The compositions under analysis are studied in an *in vitro* model which mimics this situation, i.e. human endothelial cells exposed to hypoxic conditions. Two important steps of activation of endothelial cells incubated for 120 minutes under hypoxia are tested: (1) decrease in ATP content, which is the starting point of the activation cascade, and (2) increase in the activity of phospholipase A2, an enzyme responsible for the release of precursors of inflammatory mediators.

Hypoxia-activated endothelial cells also increase the adhesiveness thereof to neutrophils.

Only composition 1 was tested in said *in vitro* model of CVI to evaluate the ATP content and the expression of phospholipase A2 (PLA2) on the hypoxia-induced activation of human umbilical vein endothelial cells (HUVEC).

### 2.2.1 Experimental design and material

Human umbilical vein / vascular endothelial cells (HUV-EC-C [HUVEC] (ATCC^{®} CRL-1730 ^{™}) were obtained from the American Type Culture Collection (ATCC) and cultured in DMEM supplemented with 10% FBS, 100 units/ml penicillin, 100 µg/ml streptomycin and kept in a Thermo carbon dioxide incubator (Thermo Fisher Scientific, Waltham, MA, USA) at 37°C with a humidified atmosphere at 95% air and 5% CO₂. The experimental design included a 96-well plate containing a sub-confluent cell monolayer (about 88% confluence) divided into the following groups:
1) Control group under normoxia conditions (n = 9): 150 µl (for each well) of complete medium under normoxia;
2) Control group + compound under normoxia conditions (n = 9): 150 µl (for each well) of compound at the concentration of 200 mg / ml dissolved in complete medium under normoxia;
3) Control group under hypoxia condition (n = 9): 150 µl (for each well) of complete medium under hypoxia;
4) Control + compound under hypoxia conditions (n = 9): 150 µl (for each well) of compound at the concentration of 200 mg / ml dissolved in complete medium under hypoxia.

### 2.2.2. Treatment

HUVECs (4 × 104/well) were plated on 96-well microtiter plates up to a final volume of 150 µl. After 24 hours of adhesion at 37°C and 5% COz, the cells were exposed under hypoxia conditions (100% N) at 37°C. For incubation, the cells were rinsed twice with HBSS and covered with 0.7 ml of HBSS. The medium was reduced to a uniform thin layer to decrease the diffusion distances of the atmospheric gases. Hypoxia was produced with a 100% N atmosphere in a gas chamber of the incubator. PO was 130 mmHg under normal conditions, decreasing to 10 mmHg after 30 minutes of hypoxia. One hundred and twenty (120) minutes of hypoxia were opted for because it is the maximum hypoxia time that endothelial cells can sustain without loss of viability. The corresponding controls were always performed using cells incubated under the same conditions but maintained under normoxia. The compound was tested for 120 minutes under normoxia and hypoxia conditions.

### 2.2.3. Evaluation of the ATP content

The ATP test was performed using an ATP bioluminescence assay kit (MAK190, Sigma) using luciferase and luciferin. To ensure reproducibility and low background, all technical precautions described in the corresponding manual sheet were observed. After incubation under normoxia or hypoxia, HUVECs were rinsed with PBS, lysed with 0.5 ml of somatic cell ATP releasing reagent for a few seconds and the supernatant recovered for analysis conducted in a luminometer (Bio-counter M2010). The absolute ATP values obtained from the experiments were expressed in RLU (relative light units) / mg of proteins. The amount of proteins was analysed for each assay to correct the amount of ATP measured in the bioluminescence assay with the number of cells present in each well.

### 2.2.4. Evaluation of the expression of phospholipase A2 (PLA2)

After incubation under normoxia or hypoxia, the cells were recovered in 100 ml of lysate buffer (Tris 20 mM, KCl 150 mM, EDTA 1 mM, Triton X-100 0.05%, pH 7.5, protease and phosphatase inhibitors) at 48. PLA2 activity was assayed using diheptanoyl-thio-phosphatidylcoline as substrate. The activity of cPLA2 was analysed using arachidonoyl thio-phosphatidyllcholine as substrate in the presence of BEL to inhibit iPLA2. After hydrolysis of the thioester linked to the sn-2 position, the free thiols are detected using 5.50-dithiobis-2-nitrobenzoic acid (DTNB) at 405 nm. The activities were calculated in mmol of substrate / min using a DNTB coefficient of extinction of 10.66 mM^1 and they refer to mg of proteins assayed using the Bradford method.

### 2.3 Ex vivo preparation of mesenteric vessels: contraction studies

Mesenteric vessels are removed from mice and placed in an oxygenated Krebs solution (95% Oz, 5% COz). The Krebs buffer has the following composition (mM): 137 Na +, 5.89 K +, 2,50 Ca₂ +, 1.20 Mg₂ +, 134 Cl⁻, 18.0 HCO₃⁻, 1.20 H₂PO₄⁻ and 5.60 glucose, gassed with 95% Oz + 5% COz and maintained at 37°C. The samples are thoroughly rinsed using adipose tissue and cut into 4-6 mm rings. The rings are cut and opened, forming arterial or venous strips. The strips are mounted between a fixed point in the organ chambers containing the Krebs buffer and a Grass FT 03 strain gauge transducer connected to a Grass polygraph (Grass Instrument Inc., Quincy, MA, USA), allowing continuous measurement of auxometric tension. The strips will be mechanically stretched (2-2.5 g for arteries and 3.5-4.0 g for veins) and allowed to balance for 45-60 minutes, resulting in optimal passive tension for vessels (0.5-1.0 for arteries and 1.0-1.5 for veins). After replacing the Krebs buffer, the extract of the composition under examination is added cumulatively in the concentration range from 0.1-10 mg / ml. After stimulation with the composition under examination, the strips are washed several times and then the maximum contraction is induced by replacing the Krebs buffer with a high concentration Krebs buffer K⁺ (128 mM K⁺, 15.4 mM Na⁺ and other ions as described above). Then the contraction of the veins is measured.

### 2.4 In vivo studies: damage to the ligated saphenous veins

Male NMRI mice (12 weeks) are housed in wire cages under controlled climatic conditions (with humidity of 50 ± 10% and 20-22°C), fed with standard laboratory food and allowed to water *ad libitum.* Animal experiments comply with Italian regulations on the protection of animals used for experimental and other scientific purposes (Italian Ministerial Decree No. 116192) and with EU regulations (OJ L 358/1 of 12/18/1986). The animals used for this study are randomly selected from the appropriate ones, available at that time.

Mice are divided into six experimental groups:
- group 1 receives the carrier (PBS) without the composition under examination (n = 8);
- group 2 receives the composition under examination (composition 1) for 7 days (n = 8);
- group 3 receives Simvastatin (a statin) for 7 days (n = 8);
- group 4 is subjected to saphenous vein ligation for 7 days (n = 8);
- group 5 is subjected to saphenous vein ligation and receives the composition under examination for 7 days (n = 8);
- group 6 is subjected to saphenous vein ligation and receives Simvastatin for 7 days (n = 8).

The compound under analysis (composition 1) was administered at a dose of 170 mg / kg corresponding to 864 mg / day, which represent the dose corresponding to the recommended amount of this compound for humans. Furthermore, the mice were treated with Simvastatin because statins are a potential pharmacological treatment option suitable to prevent the growth of varicose veins and to limit the formation of relapses after varicose vein surgery Based on the weight of the animal and ingestion, the dose of Simvastatin applied was 20 mg / kg per day.

### 2.4.1 STAGE I: Induction

The mice were acclimated for 7 days before starting the experiments and they are divided into 6 groups of eight mice each. The underlying vascularisation of a posterior limb is surgically exposed and the saphenous vein is ligated into a region distal from its origin. Normal control groups are treated with PBS (phosphate buffered saline) without damage to the saphenous vein ligation. Seven days after surgery mice are sacrificed under anaesthesia and the lateral veins (saphenous) were removed and processed for histological and immunohistochemical studies.

### 2.4.2 Histopathological examination

The saphenous veins, collected and fixed in formalin, are post-fixed in 50 mM (pH 7.4) phosphate buffer containing 4% paraformaldehyde overnight and then immersed in 30% sucrose solution (in 50 mM phosphate-buffered saline). Frozen samples are sectioned at 30 µm using a cryostat, haematoxylin and eosin stained (H&E) and then evaluated under an optical microscope.

### 2.4.3 Immunohistochemistry examination for VEGF (vascular endothelial grow factor)

Saphenous veins are collected and repaired in formalin. Cryosections (10 microns thick) are blocked in 5% normal goat serum, 1% bovine serum albumin without permeabilization and stained with primary antibodies: Anti-mouse VEGF (Santa Cruz Biotechnology) followed by secondary antibodies labelled with Alexa-Fluor (Molecular probes). The images are acquired under a Leica microscope using Axio Scan.Z1 (Zeiss). Images are assessed using Fiji / imaged.

### 2.4.4. Evaluation of IL-1β and TNF-α cytokines

The inflammatory process is crucial for the development of incompetent valves and for the remodelling of the venous wall. The role of inflammation in this CVI model was studied through cytokine quantification using ELISA kit assay.

### 3. Permeation studies on Franz diffusion cells

The vertical diffusion cell or Franz diffusion cell is a simple and reproducible test for measuring drug release of drugs from tablets, creams, powders, ointments, gels, etc. which is emerging as the preferred apparatus for *in vitro* drug release testing.

The cell comprises two parts: (a) the donor chamber containing the composition to be tested and (b) the receptor chamber containing the receptor medium.

The rat jejunal mucosa is fixed between the donor and the receptor chamber designed to act as a diffusion conduit and serving to contain the composition under examination, while ensuring that it remains in contact with the receptor medium. The receptor chamber is filled with 1 ml of physiological buffer and stabilised for 30 minutes. The receptor fluid is mixed with a magnetic stirring bar (200 rpm), while the temperature is maintained at 32±2°C. The composition under examination is applied to the upper part of the mucous membranes. All openings, including the upper part of the donor and the receptor arm, were occluded with parafilm to prevent evaporation. During the membrane diffusion study, permeation detection samples are collected over a period of time comprised from 0.5 hours to 24 hours and analysed by means of UV analysis.

The flow indicates the permeation rate, which can be calculated from the amount of permeation material per time and area, with respect to the initial concentration applied to the upper area of the tissue and the unit is µg / h / cm². The slope of the line (regression) represents the product release rate.

Both composition 1 (according to the invention) and composition 2 (comparison) were tested in the permeation model on Franz cells.

### 3. RESULTS

### 3.1 Permeation studies on Franz diffusion cells

The diffusion test on composition 1 (with microencapsulation) and composition 2 (without microencapsulation) through the intestinal mucosa was classified based on the diffusion rate of the compounds over 24 hours. Composition 1 (with microencapsulation) showed a diffusion rate visible from the first hour to the fourth hour (0.005%), through the Franz diffusion cell apparatus over time up to 24 hours (line with circles, Figure 2). Composition 2 (without microencapsulation) showed a minimal and negligible absorption rate (0.0001%), visible only at the first hour (line with squares, Figure 2).

### 3.2. In vitro study of ATP and PLA 2 evaluation in HUVECs

Previous results have shown that endothelial cells can be strongly activated by hypoxia, a condition that mimics the *in vivo* decrease in oxygen supply during venous insufficiency events. This activation is due to a decrease in the ATP content of the cell, which leads to an increase in cytosolic calcium concentration and to the activation of an inflammatory condition.

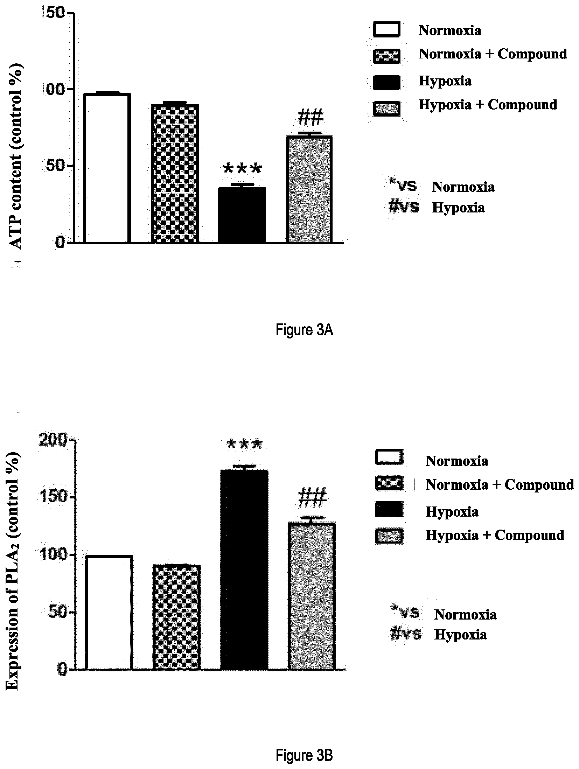

In this study, when HUVECs were exposed to hypoxia for 120 minutes, their ATP content decreased from about 45% of the control cell value, maintained under normoxia conditions (Fig. 3A).

The ATP level decreased linearly during the overall incubation, under hypoxia conditions (Figure 2A). However, exposure with the compound under analysis (composition 1), at the concentration of 0.2 g/mol, significantly inhibited the hypoxia-induced decrease of ATP content in endothelial cells. As a matter of fact, significant protection of 68% was observed when HUVECs were stimulated with the above compound for 120 minutes (Figure 3A).

Furthermore, in the vascular system, prostaglandin synthesis (PG) is important to maintain normal haemostasis and vascular tone. Endothelial functions, including the release of PG, are regulated by changes in endothelium functions during ischemia processes and under a variety of pathological conditions, including myocardial or cerebral infarction, thrombosis and venous insufficiency. It has been shown that the decrease in oxygen tension (hypoxia) activates endothelial cells leading to an increase in PGs, resulting in an increase in the expression of PLA2 in the human umbilical vein endothelial cells. In this study, the increase in hypoxia-induced expression of PLA2 was reduced by treatment with the compound under analysis (composition 1) (Figure 3B, concentration of 0.2 g / ml).

The data represent the result of three independent experiments. One way ANOVA test followed by Bonferroni post hoc test. (A and B) *** p <0.001 with respect to the control under normoxia; ## p <0.01 with respect to the control under hypoxia.

### 3.3 Ex vivo study on mesenteric vessels: contraction analysis

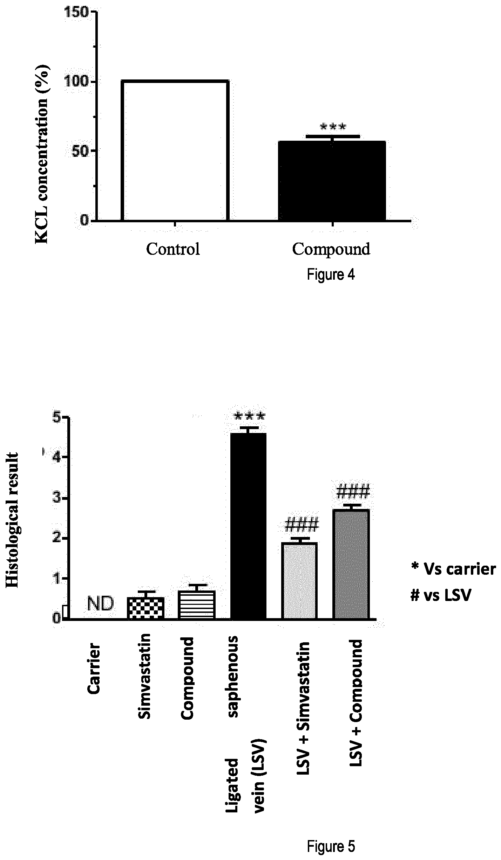

Since it is known that the depolarization of the membrane by KCl stimulates the entry of Ca²⁺ through voltage-dependent Ca2 channels (probably activating the Ca²⁺ sensitization pathways), in order to better understand the CA²⁺ dependent contractile response of this compound, the venous contractile action of the compound under analysis upon membrane depolarization was tested, by means of 96 mM of KCl (Figure 4). In the mesenteric vein segments, pre-treated with the compound under analysis (10"4 M) for 2 hours, the KCL-induced contraction was significantly reduced compared to the control group (untreated) (Figure 4), suggesting that our compound acted improving venous tone. The data represent the mean of three independent experiments. Paired t-test *** p <0.001 with respect to the control.

### 3.4 In vivo studies: damage to the ligated saphenous veins (LSV)

### 3.4.1. Histopathological examination

Histopathological examination of the lateral saphenous vein ligated for 7 days revealed inflammatory cellular infiltration, structural change of the tunica adventitia and formation of focal epithelial oedema, with the collapse of the entire vessel wall with respect to the control group (Figure 5; microscope images not shown). In control mice, treated with Simvastatin or with the compound, for 7 days, no significant changes in the lumen of the vessel were observed, just like there were no signs of tissue inflammation (Figure 5; microscope images not shown). The treatment with the aforementioned compound, at a dose of 170 mg / kg, for 7 days significantly reduced the neutrophil accumulation, the hypertrophy of the tunica media, reducing the collapse of the wall caused by the ligation, thanks to its venotonic action thereof (Fig. 5) (One way ANOVA test followed by Bonferroni post hoc test. ND, not detectable; *** p <0.001 with respect to carrier and ### p <0.001 with respect to LSV.).

### 3.4.2. Immunohistochemistry examination for VEGF (vascular endothelium growth factor)

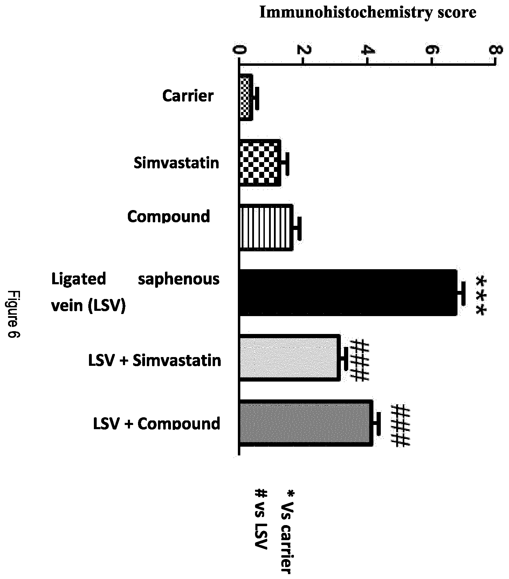

VEGF is a factor involved in vascular integrity; in particular, primary varicose veins show aberrant release of VEGF. In this CVI model, the variation of VEGF was examined by means of immunohistochemistry analysis. Positive staining for VEGF was significantly increased in tunica media and adventitia of the vessel of the ligated saphenous vein with respect to the non-ligated saphenous veins (Figure 6). Oral treatment with the compound under analysis (170 mg / kg) significantly reduced the positive staining for VEGF (Figure 6), underlining the ability of the compound according to the invention (composition 1) to decrease vascular permeability, a critical event in inflammation and angiogenesis.

The data represent the mean of three independent experiments. One way ANOVA test followed by Bonferroni post hoc test (ND, not detectable; *** p <0.001 with respect to carrier, ### p <0.001 with respect to LSV).

### 3.4.3. Evaluation of cytokines

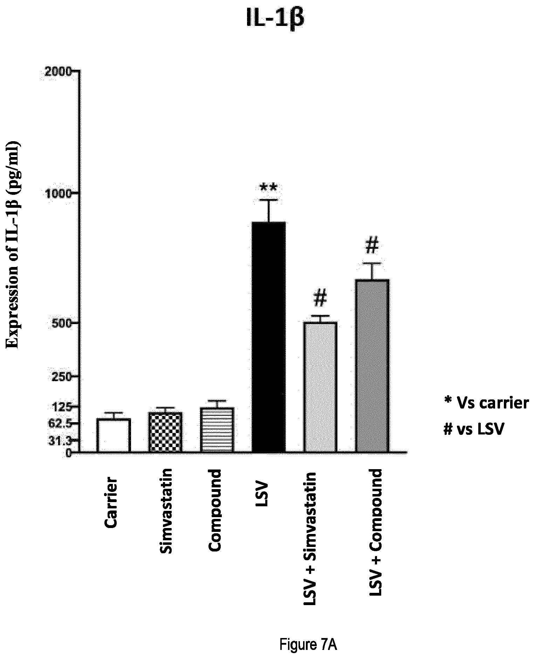

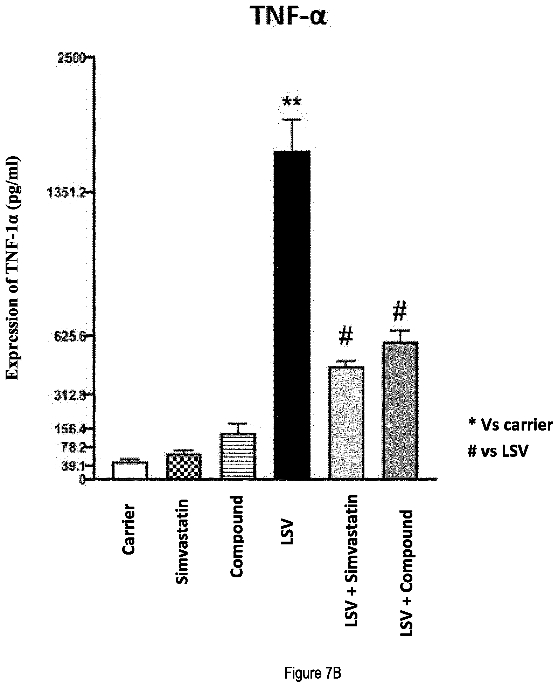

Quantification of IL-1β and TNF-α was significantly increased in the vessel of the ligation-induced damage to the saphenous vein with respect to the control veins (Figure 7A and 7B, respectively). Evaluation of the cytokines of the saphenous veins treated with Simvastatin or with the compound under analysis according to the invention (composition 1) showed a significant decrease in the quantification of IL-1β and TNF-α (Fig. 7A and 7B), highlighting the ability of the compound of the invention to decrease inflammation.

## Claims

1. A microencapsulated formulation with a coating matrix,
wherein said formulation comprises or, alternatively, consists of:
(a) a diosmin;
(b) a troxerutin;
(c) coumarins or an extract of a plant species comprising coumarins; and
wherein said coating matrix comprises or, alternatively, consists of at least one lipid.

2. The formulation according to claim 1, wherein said at least one lipid is a phospholipid and, optionally, said coating matrix further comprises at least one acceptable pharmaceutical or food grade additive and/or excipient; preferably
said at least one lipid is a phosphatidylcholine or a lecithin (E322); preferably a sunflower or corn or soy lecithin (E322); more preferably a sunflower or corn or soy lecithin (E322).

3. The formulation according to any one of claims 1-2, wherein said formulation has a structure comprising or, alternatively, consisting of said coating matrix comprising or, alternatively, consisting of at least one lipid in which core-shell structures are dispersed,
wherein said shell comprises or, alternatively, consists of (a) diosmin; (b) troxerutin; and (c) coumarins or an extract of a plant species comprising coumarins, and
wherein said core comprises or, alternatively, consists of said coating matrix comprising or, alternatively, consisting of at least one lipid; preferably
said microencapsulated formulation has an average particle diameter comprised from 100 µm to 500 µm, preferably from 150 µm to 400 µm.

4. The formulation according to any one of claims 1-3, wherein said (a) diosmin consists of micronized diosmin; preferably micronized diosmin having an average particle size comprised in the range from 0.5 µm to 10 µm; more preferably diosmin obtained from hesperidin, micronized and comprising a buffer system; preferably
wherein said (b) troxerutin consists of (b.i) an extract of *Styphnolobium japonicum* titrated in troxerutin in a percentage by weight with respect to the total weight of the extract comprised in the range from 60% to 99.5%, preferably from 70% to 98%, or (b.ii) a troxerutin obtained by synthetic route; preferably
wherein said (c) coumarins or an extractof a plant species comprising coumarins consists of (c.i) an extract of European horse chestnut, *Aesculus hippocastanum* L., comprising total coumarins in a percentage by weight comprised in the range from 1% to 40% with respect to the total weight of the extract, preferably from 10% to 30%.

5. The formulation according to any one of claims 1-4, wherein said formulation comprises or, alternatively, consists of:
(a) micronized diosmin having an average particle size comprised in the range from 0.5 µm to 10 µm; preferably diosmin obtained from hesperidin, micronized and comprising a buffer system;
(b) troxerutin, wherein said (b) troxerutin is (b.i) an extract of *Styphnolobium japonicum* titrated in troxerutin in a percentage by weight with respect to the total weight of the extract comprised in the range from 60% to 99.5% or (b.ii) troxerutin obtained by synthetic route;
(c.i) an extract of European horse chestnut, *Aesculus hippocastanum* L., comprising total coumarins in a percentage by weight comprised in the range from 1% to 40% with respect to the total weight of the extract;
and wherein said coating matrix comprises or, alternatively, consists of a phosphatidylcholine or lecithin (E322), preferably sunflower lecithin (E322), and, optionally, at least one acceptable pharmaceutical or food grade additive and/or excipient.

6. A composition comprising:
- the microencapsulated formulation according to any one of claims 1-5;
and, optionally,
- at least one acceptable pharmaceutical or food grade additive and/or excipient.

7. The composition according to claim 6 besides said microencapsulated formulation according to any one of claims 1-5, said composition further comprises vitamin C; preferably
said composition is formulated for oral use or for topical skin use or for topical rectal use; wherein said composition is formulated for topical use; preferably for topical skin use or for topical rectal use.

8. The formulation or the composition according to any one of claims 1-7 for use as medicament; preferably
for use as vasoactive agents; preferably, for use in a method for the preventive and/or curative treatment of a disorder of the circulatory system, in a subject in need.

9. The formulation or the composition according to any one of claims 1-7 for use in a method for the preventive and/or curative treatment of a chronic venous insufficiency or chronic venous disease, and of diseases or symptoms related to or deriving from said chronic venous insufficiency, in a subject in need; preferably
wherein said diseases or symptoms related to or derived from said chronic venous insufficiency are selected from: oedema, difficulty in walking, burning sensation, itchy skin, paraesthesia or formication, pain, muscle cramps, sensation of bloating, sensation of heaviness in the lower limbs, restless legs syndrome, fatigue in the legs, postphlebitic syndrome, stasis dermatitis and couperose skin.

10. The formulation or the composition according to any one of claims 1- 7, for use in a method for the preventive and/or curative treatment of a haemorrhoidal disease or edematous fibrosclerotic panniculopathy (PEFS or cellulite) or lymphedema or trauma or endothelial dysfunction, and of diseases or symptoms related thereto to or deriving therefrom, in a subject in need.

11. The cosmetic use of the formulation or composition according to any one of claims 1-7 to maintain or improve skin tone, to reduce or eliminate telangiectasias or couperose skin or xerosis cutis.

12. A process for the preparation of a microencapsulated formulation with a coating matrix comprising or, alternatively, consisting of a lipid according to any one of claims 1-5, wherein said process provides for the use of a fluid bed chamber.

## Patentansprüche

1. Mikroverkapselte Formulierung mit einer Beschichtungsmatrix,
wobei die Formulierung Folgendes umfasst oder alternativ daraus besteht:
(a) ein(em) Diosmin;
(b) ein(em) Troxoerutin;
(c) Coumarine(n) oder ein(en/em) Extrakt einer Pflanzenspezies, umfassend Coumarine; und
wobei die Beschichtungsmatrix wenigstens ein Lipid umfasst oder alternativ daraus besteht.

2. Formulierung gemäß Anspruch 1, wobei das wenigstens eine Lipid ein Phospholipid ist und die Beschichtungsmatrix optional weiterhin wenigstens ein(en) akzeptable(s/n) pharmazeutisches oder Nahrungsmitteladditiv und/oder Hilfsstoff umfasst; bevorzugt
das wenigstens eine Lipid ein Phosphatidylcholin oder ein Lecithin (E322) ist; bevorzugt ein Sonnenblumen- oder Mais- oder Sojalecithin (E322); bevorzugter ein Sonnenblumen- oder Mais- oder Sojalecithin (E322).

3. Formulierung gemäß irgendeinem der Ansprüche 1-2, wobei die Formulierung eine Struktur hat, umfassend die oder alternativ bestehend aus der Beschichtungsmatrix, umfassend ein oder alternativ bestehend aus wenigstens einem Lipid, in dem Kern-Schale-Strukturen dispergiert sind,
wobei die Schale umfasst oder alternativ besteht aus (a) Diosmin, (b) Troxoerutin; und (c) Coumarine(n) oder ein(em) Extrakt einer Pflanzenspezies, umfassend Coumarine, und
wobei der Kern die Beschichtungsmatrix umfasst oder alternativ daraus besteht, umfassend ein oder alternativ bestehend aus wenigstens einem Lipid; wobei bevorzugt
die mikroverkapselte Formulierung einen durchschnittlichen Partikeldurchmesser von 100 µm bis 500 pm, bevorzugt von 150 µm bis 400 µm, hat.

4. Formulierung gemäß irgendeinem der Ansprüche 1-3, wobei das (a) Diosmin aus mikronisiertem Diosmin besteht; bevorzugt mikronisiertem Diosmin mit einer durchschnittlichen Partikelgröße im Bereich von 0,5 µm bis 10 µm; bevorzugter von Hesperidin erhaltenem Diosmin, mikronisiert und ein Puffersystem umfassend; bevorzugt
wobei das (b) Troxoerutin besteht aus: (b.i) einem Extrakt von *Styphnolobium japonicum,* titriert in Troxoerutin in einem Gewichtsprozentsatz in Bezug auf das Gesamtgewicht des Extrakts im Bereich von 60% bis 99,5%, bevorzugt von 70% bis 98%, oder (b.ii) einem auf synthetischem Weg erhaltenen Troxoerutin; wobei bevorzugt (c) die Coumarine oder ein Extrakt einer Pflanzenspezies, umfassend Coumarine, bestehen aus: (c.i) einem Extrakt von Europäischer Rosskastanie, *Aesculus hippocastanum* L., umfassend Gesamtcoumarine in einem Gewichtsprozentsatz im Bereich von 1% bis 40% in Bezug auf das Gesamtgewicht des Extrakts, bevorzugt von 10% bis 30%.

5. Formulierung gemäß irgendeinem der Ansprüche 1-4, wobei die Formulierung umfasst oder alternativ besteht aus:
(a) mikronisierte(s/m) Diosmin mit einer durchschnittlichen Partikelgröße im Bereich von 0,5 µm bis 10 µm; bevorzugt von Hesperidin erhaltene(s/m) Diosmin, mikronisiert und ein Puffersystem umfassend;
(b) Troxoerutin, wobei das (b) Troxoerutin (b.i) ein Extrakt von *Styphnolobium japonicum,* titriert in Troxoerutin in einem Gewichtsprozentsatz in Bezug auf das Gesamtgewicht des Extrakts im Bereich von 60% bis 99,5%, ist, oder (b.ii) auf synthetischem Weg erhaltenes Troxoerutin ist;
(c.i) eine(n/m) Extrakt von Europäischer Rosskastanie, *Aesculus hippocastanum* L., umfassend Gesamtcoumarine in einem Gewichtsprozentsatz im Bereich von 1% bis 40% in Bezug auf das Gesamtgewicht des Extrakts;
und wobei die Beschichtungsmatrix umfasst oder alternativ besteht aus: ein(em) Phosphatidylcholin oder Lecithin (E322), bevorzugt Sonnenblumenlecithin (E322) und optional wenigstens ein(en/em) akzeptablen pharmazeutischen oder Nahrungsmitteladditiv und/oder Hilfsstoff.

6. Zusammensetzung, umfassend:
- die mikroverkapselte Formulierung gemäß irgendeinem der Ansprüche 1-5;
und optional
- wenigstens ein(en) akzeptable(s/n) pharmazeutisches oder Nahrungsmitteladditiv und/oder Hilfsstoff.

7. Zusammensetzung gemäß Anspruch 6, wobei die Zusammensetzung neben der mikroverkapselten Formulierung gemäß irgendeinem der Ansprüche 1-5 weiterhin Vitamin C umfasst; wobei bevorzugt
die Zusammensetzung für die orale Anwendung oder für die topische Hautanwendung oder für die topische rektale Anwendung formuliert ist; wobei die Zusammensetzung für die topische Anwendung formuliert ist; bevorzugt für die topische Hautanwendung oder die topische rektale Anwendung.

8. Formulierung oder Zusammensetzung gemäß irgendeinem der Ansprüche 1-7 zur Verwendung als Medikament; bevorzugt
zur Verwendung als vasoaktive Agenzien; bevorzugt zur Verwendung in einem Verfahren zur präventiven und/oder kurativen Behandlung einer Störung des Kreislaufsystems in einem bedürfenden Subjekt.

9. Formulierung oder Zusammensetzung gemäß irgendeinem der Ansprüche 1-7 zur Verwendung in einem Verfahren zur präventiven und/oder kurativen Behandlung einer chronischen Veneninsuffizienz oder einer chronischen Venenerkrankung und von Erkrankungen oder Symptomen im Zusammenhang mit oder sich ableitend aus der chronischen Veneninsuffizienz in einem bedürfenden Subjekt; bevorzugt
wobei die Erkrankungen oder Symptome im Zusammenhang mit oder sich ableitend aus der chronischen Veneninsuffizienz ausgewählt sind aus: Ödem, Schwierigkeiten beim Gehen, brennendem Gefühl, Hautjucken, Parästhesie oder Ameisenlaufen, Schmerzen, Muskelkrämpfen, Aufblähungsgefühl, Schweregefühl in den unteren Gliedmaßen, Restless-Legs-Syndrom, Müdigkeit in den Beinen, postthrombotischem Syndrom, Stauungsekzem und Couperose.

10. Formulierung oder Zusammensetzung gemäß irgendeinem der Ansprüche 1-7 zur Verwendung in einem Verfahren zur präventiven und/oder kurativen Behandlung einer Hämorrhoidenerkrankung oder von ödematöser fibrosklerotischer Pannikulopathie (PEFS oder Cellulite) oder Lymphödem oder Trauma oder Endotheldysfunktion und von damit in Zusammenhang stehenden oder sich daraus ableitenden Erkrankungen und Symptomen in einem bedürfenden Subjekt.

11. Kosmetische Anwendung der Formulierung oder Zusammensetzung gemäß irgendeinem der Ansprüche 1-7 zum Aufrechterhalten oder Verbessern des Hauttonus, zum Reduzieren oder Eliminieren von Teleangiektasien oder Couperose oder Xerosis cutis.

12. Verfahren zur Herstellung einer mikroverkapselten Formulierung mit einer Beschichtungsmatrix, umfassend ein oder alternativ bestehend aus einem Lipid gemäß irgendeinem der Ansprüche 1-5, wobei das Verfahren die Verwendung einer Wirbelbettkammer bereitstellt.

## Revendications

1. Formulation microencapsulée avec une matrice d'enrobage, laquelle formulation comprend ou, en variante, consiste en :
(a) une diosmine ;
(b) une troxérutine ;
(c) des coumarines ou un extrait d'une espèce végétale comprenant des coumarines ; et
dans laquelle ladite matrice d'enrobage comprend ou, en variante, consiste en, au moins un lipide.

2. Formulation selon la revendication 1, dans laquelle ledit au moins un lipide est un phospholipide et, éventuellement, ladite matrice d'enrobage comprend en outre au moins un excipient et/ou additif de qualité pharmaceutique ou alimentaire acceptable ; de préférence
ledit au moins un lipide est une phosphatidylcholine ou une lécithine (E322) ; de préférence une lécithine de tournesol ou de maïs ou de soja (E322) ; mieux encore une lécithine de tournesol ou de maïs ou de soja (E322).

3. Formulation selon l'une quelconque des revendications 1 et 2, laquelle formulation a une structure comprenant ou, en variante, consistant en, ladite matrice d'enrobage comprenant ou, en variante, consistant en, au moins un lipide dans lequel des structures coeur-gaine sont dispersées,
dans laquelle ladite gaine comprend ou, en variante, consiste en, (a) diosmine ; (b) troxérutine ; et (c) coumarines ou un extrait d'une espèce végétale comprenant des coumarines, et
dans laquelle ledit coeur comprend ou, en variante, consiste en, ladite matrice d'enrobage comprenant ou, en variante, consistant en, au moins un lipide ; de préférence
laquelle formulation microencapsulée a une granulométrie moyenne comprise entre 100 µm et 500 µm, de préférence entre 150 µm et 400 µm.

4. Formulation selon l'une quelconque des revendications 1 à 3, dans laquelle ladite diosmine (a) consiste en diosmine micronisée ; de préférence en diosmine micronisée ayant une granulométrie moyenne comprise dans la plage allant de 0,5 µm à 10 µm ; mieux encore en diosmine obtenue à partir d'hespéridine, micronisée et comprenant un système tampon ; de préférence
dans laquelle ladite troxérutine (b) consiste en (b.i) un extrait de *Styphnolobium japonicum* titrée en troxérutine en un pourcentage en poids, par rapport au poids total de l'extrait, compris dans la plage allant de 60 % à 99,5 %, de préférence de 70 % à 98 %, ou (b.ii) une troxérutine obtenue par voie de synthèse ; de préférence
dans laquelle lesdites coumarines (c) ou un extrait d'une espèce végétale comprenant des coumarines consistent en (c.i) un extrait de marron d'inde, *Aesculus hippocastanum* L., comprenant des coumarines totales en un pourcentage en poids compris dans la plage allant de 1 % à 40 % par rapport au poids total de l'extrait, de préférence de 10 % à 30 %.

5. Formulation selon l'une quelconque des revendications 1 à 4, laquelle formulation comprend ou, en variante, consiste en :
(a) diosmine micronisée ayant une granulométrie moyenne comprise dans la plage allant de 0,5 µm à 10 µm ; de préférence diosmine obtenue à partir d'hespéridine, micronisée et comprenant un système tampon ;
(b) troxérutine, laquelle troxérutine (b) est (b.i) un extrait de *Styphnolobium japonicum* titrée en troxérutine en un pourcentage en poids, par rapport au poids total de l'extrait, compris dans la plage allant de 60 % à 99,5 % ou (b.ii) une troxérutine obtenue par voie de synthèse ;
(c) un extrait de marron d'inde, *Aesculus hippocastanum* L., comprenant des coumarines totales en un pourcentage en poids compris dans la plage allant de 1 % à 40 % par rapport au poids total de l'extrait ;
et dans laquelle ladite matrice d'enrobage comprend ou, en variante, consiste en, une phosphatidylcholine ou lécithine (E322), de préférence une lécithine de tournesol (E322), et éventuellement au moins un excipient et/ou additif de qualité pharmaceutique ou alimentaire acceptable.

6. Composition comprenant :
- la formulation microencapsulée selon l'une quelconque des revendications 1 à 5 ; et éventuellement
- au moins un excipient et/ou additif de qualité pharmaceutique ou alimentaire acceptable.

7. Composition selon la revendication 6 comprenant, outre ladite formulation microencapsulée selon l'une quelconque des revendications 1 à 5, de la vitamine C ; de préférence
laquelle composition est formulée pour un usage oral ou un usage topique cutané ou un usage topique rectal ; laquelle formulation est formulée pour un usage topique ; de préférence pour un usage topique cutané ou pour un usage topique rectal.

8. Formulation ou composition selon l'une quelconque des revendications 1 à 7, pour une utilisation en tant que médicament ; de préférence
pour une utilisation en tant qu'agent vasoactif ; de préférence pour une utilisation dans une méthode de traitement préventif et/ou curatif d'un trouble du système circulatoire chez un sujet en ayant besoin.

9. Formulation ou composition selon l'une quelconque des revendications 1 à 7, pour une utilisation dans une méthode de traitement préventif et/ou curatif d'une insuffisance veineuse chronique ou d'une maladie veineuse chronique, et de maladies ou symptômes liés à ou dérivant de ladite insuffisance veineuse chronique, chez un sujet en ayant besoin ; de préférence
dans laquelle lesdites maladies ou lesdits symptômes liés à ou dérivant de ladite insuffisance veineuse chronique sont choisis parmi : un oedème, des difficultés à marcher, une sensation de brûlure, des démangeaisons cutanées, une paresthésie ou des fourmillements, une douleur, des crampes musculaires, une sensation de gonflement, une sensation de lourdeur des membres inférieurs, un syndrome des jambes sans repos, une fatigue des jambes, un syndrome post-phlébitique, une dermite de stase, et une peau couperosée.

10. Formulation ou composition selon l'une quelconque des revendications 1 à 7, pour une utilisation dans une méthode de traitement préventif et/ou curatif d'une maladie hémorroïdale ou d'une panniculopathie oedomato-fibro-sclérotique (PEFS ou cellulite) ou d'un lymphoedème ou d'un traumatisme ou d'un dysfonctionnement endothélial, et de maladies et symptômes liés à ou dérivant d'une telle maladie, chez un sujet en ayant besoin.

11. Utilisation cosmétique de la formulation ou composition selon l'une quelconque des revendications 1 à 7 pour maintenir ou améliorer le tonus cutané, pour réduire ou éliminer une télangiectasie ou une peau couperosée ou une xérose cutanée.

12. Procédé pour la préparation d'une formulation microencapsulée avec une matrice d'enrobage comprenant ou, en variante, consistant en, un lipide, selon l'une quelconque des revendications 1 à 5, lequel procédé permet l'utilisation d'une chambre à lit fluide.
